# EUROPÄISCHE PATENTANMELDUNG

(11) **EP 2 438 860 A1**
(43) Veröffentlichungstag der Anmeldung: **11.04.2012**
(21) Anmeldenummer: 11183752.2
(22) Anmeldetag: 04.10.2011
(51) Int. Cl.: A61B 5/1473, A61B 5/145, B01D 69/14, B01D 71/06, F16K 99/00, F15C 1/00

(54) **Medizinisches Sensorsystem**

(30) Priorität: 07.10.2010 US 390621 P; 12.11.2010 US 412802 P; 24.08.2011 US 526695 P
(71) Anmelder: BIOTRONIK SE & Co. KG, 12359 Berlin (DE)
(72) Erfinder: Bunge, Andreas, 04105 Leipzig (DE); Biela, Sarah, 12053 Berlin (DE); Bode, Sven, 10829 Berlin (DE); Nähring, Jörg, 52066 Aachen (DE); Trieu, Hoc Khiem, 21394 Westergellersen (DE); Urban, Gerald, 79104 Freiburg (DE)
(74) Vertreter: Lindner-Vogt, Karin L.

(57) **Zusammenfassung**

Die Erfindung geht aus von einem medizinischen Sensorsystem (10, 10') zum Nachweis von zumindest einem Merkmal (12) eines tierischen und/oder menschlichen Körpers, mit zumindest einem Sensor (14), einem ersten Merkmalsträger (16) und einem Merkmalsträgerrezeptor (18), wobei sich der erste Merkmalsträger (16) zumindest in einem Merkmalsparameter von einem zumindest zum Zeitpunkt des Nachweises vorhandenen zweiten Merkmalsträger (20) unterscheidet.

Es wird vorgeschlagen, dass der zumindest eine Sensor (14) bei dem Nachweis des zumindest einen Merkmals *in vivo* angeordnet ist.

## Beschreibung

Die Erfindung betrifft ein medizinisches Sensorsystem und ein Verfahren zum Betreiben eines solchen Systems nach den Oberbegriffen der unabhängigen Ansprüche.

Eine medizinisch interessante Klasse von Stoffen stellen Proteine dar. Diese sind im Blut beispielsweise als Enzyme, Transportmittel für andere Moleküle oder als Gerinnungsfaktoren an vielen wichtigen Vorgängen im Körper beteiligt. Es ist von großem Interesse, insbesondere im Krankheitsfall, solche Proteine oder deren Menge oder Konzentration beispielsweise im Blut zu detektieren. Hierbei werden in der Diagnostik zum Beispiel chemische, enzymatische oder optische Verfahren eingesetzt. Proteine besitzen eine durch ihre Aminosäuresequenz definierte dreidimensionale Struktur, die vom Immunsystem durch spezifische Antikörpererkennung genutzt wird, wobei Antikörper artfremde von eigenen Proteinen unterscheiden können. Diese Wechselwirkung zwischen dem Antikörper und dem so genannten Antigen kann als immunologischer Nachweis genutzt werden und hat sich als gängiges Verfahren in der *in vitro*-Diagnostik etabliert.

Auch bei einem Verdrängungsassay ("Competition Assay") wird dieses Prinzip zur Bestimmung einer Konzentration des Antigens eingesetzt. Hierbei werden die Antikörper zum Testbeginn z.B. mit den Antikörper bindenden Antagonisten versehen, wobei diese eine Fluoreszenzmarkierung tragen, wodurch ein optisches Messsignal erzeugt wird. In Gegenwart des nachzuweisenden Stoffes - einem Analyten - werden einige der fluoreszenzmarkierten Antagonisten von der Bindungsstelle der Antikörper durch den Analyten verdrängt. Hierdurch reduziert sich die Anzahl der mit Fluoreszenzmarkierung besetzten Antikörper, wodurch ein kleineres Messsignal abgeleitet wird. Die einfachste Form des Verdrängungsassays beruht auf einem gereinigten oder rekombinant erzeugten markierten Antigen, welches zusammen mit dem bindenden Antikörper als Antigen-Antikörperkomplex vorliegt. Das unmarkierte und zu messende Antigen (Analyt) bindet mit der gleichen Affinität an den Antikörper und ersetzt in gewissen Maßen das markierte Molekül. Bei bekannter Gleichgewichtskonstante der Reaktion kann man aus dem Maß des Austausches auf die vorliegende Konzentration an unmarkiertem Antigen schließen.

Ferner sind *in vitro* Verfahren ohne optisch messbare Markierungen zum Molekülnachweis bekannt. Hierbei werden z.B. Feldeffekttransistoren (FET) *in vitro* eingesetzt. Diese Ansätze beruhen darauf, dass das nachzuweisende Molekül, durch Diffusion in eine sensitive Schicht des FET gelangt. Hierfür muss das Molekül eine Ladung tragen, damit der für den Nachweis erforderliche Ladungsübertrag erfolgen kann. Alternativ können hier auf der sensitiven Schicht des FET Fängermoleküle (z.B. Antikörper) immobilisiert werden, die spezifisch den nachzuweisenden Analyten erkennen, binden und konzentrieren können. Durch die Anbindung des Analyten an die Antikörperschicht findet der Ladungsübertrag statt.

Über die letzten Jahrzehnte hinweg hat sich gezeigt, dass eine *in vitro*-Bestimmung von Analyten oft nicht ausreicht, um einen aktuellen und zeitnahen Zustand des Analyten und damit verbunden einen Zustand eines Patienten zuverlässig zu ermitteln. Insbesondere bei akuten Änderungen, beispielsweise bei chronisch Kranken, muss rasch eingegriffen werden. Hierbei ist insbesondere eine ständige Überwachung von Analyten und deren Konzentrationen über Monate oder sogar Jahre angeraten. Demgemäß besteht Bedarf an einem Sensorsystem, welches beispielsweise Analyten zuverlässig und schnell *in vivo* über einen längeren Zeitraum überwachen kann.

Keines der derzeit bekannten Sensorsysteme ist für einen *in vivo*-Einsatz anwendbar. Ein wie oben beschriebenes Antikörper- bzw. Verdrängungsassay kann üblicherweise nur einmalig in einem Nachweisverfahren verwendet werden, da die Antikörper so fest an ihr spezifisches Antigen binden, dass die ausgebildete Verbindung nicht einfach wieder gelöst werden kann. Solch ein Assay ermöglicht zwar eine hochgenaue aber nur einmalige Momentaufnahme der Konzentration des Stoffs. Für eine konzentrationsabhängige Überwachung einer Analytkonzentration über Monate/Jahre ist es jedoch erforderlich, dass die Bindung zwischen Antikörper und Analyt reversibel ist, damit der Assay nicht nur eine einmalige Momentaufnahme ermöglicht, sondern auch veränderliche Konzentrationen detektieren kann. Ferner weisen die dabei eingesetzten Antikörper keine ausgeprägte Langzeitstabilität in Kontakt mit Körperflüssigkeiten auf.

Zudem sind die optisch aktiven Marker meist gesundheitsgefährdend oder sogar toxisch, vor allem, wenn sie, beispielsweise durch Degradationsvorgänge, aus dem Sensorsystem in den Körper entweichen und dort kritische Reaktionen bzw. Entzündungsreaktionen hervorrufen können. Ferner kommt es so zu einer Verarmung an Detektionsmolekülen sowie zur Inaktivierung der optisch aktiven Marker beispielsweise durch Photobleaching, was die Funktionalität des Sensorsystems negativ beeinträchtigt. Auch sind für Antikörper-Nachweisverfahren zur Beseitigung von ungebundenen oder interferierenden Molekülen typischerweise mehrere Waschschritte notwendig. Dies würde für einen implantierbaren Sensor mit einem oben beschriebenen Nachweis eine Mikrofluidik und somit eine deutliche Vergrößerung des Implantats (inkl. Spül-Reservoir) bedeuten, was für Patienten nicht zumutbar wäre.

Bei den typischerweise mit FETs detektierten Molekülen handelt es sich um stark geladene Polyelektrolyte, wie z.B. DNA. Die meisten Proteine tragen jedoch unter physiologischen Bedingungen nur eine sehr geringe Ladung und können standardmäßig nicht mit einem FET detektiert werden oder es müssen spezielle oft gesundheitsschädliche Marker, wie Nanopartikel, zu Signalverstärkung eingesetzt werden.

Als verlässliche elektrische Nachweisverfahren für einen Zustand des Bluts konnten bisher nur pH-Sensoren und Blutgassensoren (Elektroden) etabliert werden. Benutzt werden diese Sensoren zumeist auf Intensivstationen, wobei hier ständig Zugang zu Patientenblut via Shunts und Kathetern erforderlich ist. Ein solches Sensorsystem ist nicht für einen kontinuierlichen Gebrauch über Monate oder Jahre ausgelegt.

Es ist somit kein langzeitstabiles vollständig implantierbares Sensorsystem bekannt, dass die Konzentration größerer Analyten, wie z.B. von Proteinen, überwachen und bestimmen kann, insbesondere nicht über einen Zeitraum von Monaten oder sogar Jahren. Es sind keine Ansätze bekannt, die ein vollständig implantierbares und auf Antikörper basierendes Nachweissystem für Proteine beschreiben. Bisher sind nur *in vivo* unbrauchbare *in vitro-*Systeme bekannt.

Der Erfindung liegt die Aufgabe zugrunde, ein Sensorsystem und ein Verfahren zu dessen Betrieb zur Verfügung zu stellen, das eine Überwachung eines Zustandsparameters eines menschlichen und/oder tierischen Körpers über einen langen Zeitraum bzw. über Monate oder Jahre zuverlässig, störungsfrei und kontinuierlich ermöglicht.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Die Erfindung geht aus von einem medizinischen Sensorsystem zum Nachweis von zumindest einem Merkmal zumindest eines tierischen und/oder menschlichen Körpers, mit zumindest einem Sensor, zumindest einem ersten Merkmalsträger und mit zumindest einem Merkmalsträgerrezeptor, wobei sich der erste Merkmalsträger zumindest in einem Merkmalsparameter von einem zumindest zum Zeitpunkt des Nachweises vorhandenen zweiten Merkmalsträger unterscheidet.

Es wird vorgeschlagen, dass der zumindest eine Sensor bei dem Nachweis des zumindest einen Merkmals *in vivo* angeordnet ist. Durch die erfindungsgemäße Ausgestaltung kann eine Überwachung des Merkmals besonders engmaschig erfolgen. Ferner können akute Änderungen schnell erfasst und Gegenmaßnahmen gegebenenfalls rasch ergriffen werden. Zudem ist der Nachweis schonend für den Patienten, da dieser lediglich bei der Implantation des Sensorsystems in den Körper invasiv behandelt werden muss.

In diesem Zusammenhang soll unter einem "Sensorsystem" ein System mit zumindest einem Sensor verstanden werden. Ferner kann das Sensorsystem weitere Bestandteile, wie weitere Sensoren, ein Gehäuse, Elektronikkomponenten, eine Energieversorgung, eine Telemetrieeinheit, eine Steuereinheit mit Auswerteelektronik, eine Verankerung und/oder jedes andere, dem Fachmann für einsetzbar erscheinendes Bestandteil, aufweisen.

Unter einem "Merkmal" soll ein Parameter, wie ein pH-Wert, eine Osmolalität, eine Ladung, beispielsweise eines Ions, eines Polyelektrolyten oder eines Proteins, eine Temperatur, eine Konfiguration beispielsweise einer Bindungsstelle, eine Größe, eine Masse, ein Aggregatszustand, der Wassergehalt, der Hämatokritwert, die partielle Thromboplastinzeit, die Plasma-Thrombin-Gerinnungszeit, der Quick-Wert, eine An-. bzw. Abwesenheit und/oder eine Menge eines Stoffs und/oder eines Analyten und/oder jeder andere, dem Fachmann für sinnvoll erscheinende Parameter verstanden werden. Insbesondere stellt das Merkmal eine Ladung dar und/oder ist ein Maß für eine Menge und/oder eine Konzentration eines Analyten. In diesem Zusammenhang soll unter einem "Merkmalsparameter" eine Ausprägung des Merkmals, wie sauer, alkalisch, hoch, tief, kalt, warm, heiß, stark, schwach, eine Intensität und/oder jede andere, dem Fachmann für zweckdienlich erscheinende Ausprägung verstanden werden. Dem Fachmann ist hier klar, dass ein Merkmalsparameter eines Merkmals immer relativ zu einem anderen Merkmalsparameter des Merkmals ist.

Ein Analyt stellt hier beispielsweise ein Elektrolyt, ein Fett, ein Salz, ein Ion, ein Polyelektrolyt, ein Kohlehydrat, eine Fettsäure, ein Lipid, ein Zucker, ein Nucleotid, eine Desoxyribonukleinsäure, eine Ribonukleinsäure, eine Aminosäure, ein Peptid, ein Protein, ein Antikörper, ein Hormon, ein Neurotransmitter, ein Metabolit, ein Stoffwechselprodukt, ein Antigen, einen Wirkstoffe, ein Medikament, ein Nanopartikel, ein Toxin, Wasser und/oder jeden anderen, dem Fachmann für zweckdienlich erscheinenden Stoff dar. Unter einem "Merkmal" sind auch so genannte Biomarker zu verstehen, die einen variablen Bestandteil des menschlichen oder tierischen Körpers bilden, wie beispielsweise Albumine/Globuline, Alkalische Phosphatase, Alpha-1-Globulin, Alpha-2-Globulin, Alpha-1-Antitrypsin, Alpha-1-Fetoprotein, Alpha-Amylasen, Alpha-Hydroxybutyrat-Dehydrogenase, Ammoniak, Antithrombin III, Bicarbonat, Bilirubin, Carbohydrate-Antigen 19-9, Carcino-embryonale Antigene, Chlorid, Cholesterin, Cholinesterase, Chylomikronenreste, Cobalamin/Vitamin B 12, Coeruloplasmin, C-reaktive Proteine, Cystatin C, D-Dimere, Eisen, Erythropoetin, Erythrozyten, Ferritin, Fetuin A, Fibrinogen, Folsäure/Vitamin B9, Freie Tetrajodthyronine (fT4), Freie Trijodthyronine (fT3), Gamma-Glutamyltransferase, Glukose, Glutamat-Dehydrogenase, Glutamat-Oxalazetat-Transaminase, Glutamat-Pyruvat-Transaminase, Glykohämoglobin, Hämatokrit, Hämoglobin, Haptoglobin, Harnsäure, Harnstoff, HDL-Cholesterin, Homozystein, Immunglobulin A, Immunglobulin E, Immunglobulin G, Immunglobulin M, INR, Kalium, Kalzium, Kreatinin, Kreatin-Kinase, Kupfer, Laktat, Laktat-Dehydrogenase, LDL-Cholesterin, Leukozyten, Lipase, Lipoprotein, Magnesium, korpusukuläre Hämoglobine, Myoglobin, Natrium, NT-proBNP/BNP, Phosphat, Prostataspezifische Antigene, Retikulozyten, Rheumafaktor, Thrombozyten, Thyreoidea stimulierendes Hormon, Transferrin, Triglyzeride, Troponin T, VLDL-Cholesterin.

Unter einem "Merkmal" ist auch ein "Wirkstoff" zu verstehen, wobei der Begriff "WirkStoff" typische Medikamente oder auch Methabolite umfasst, die für die Behandlung von Erkrankungen gegeben werden, wie Muskarinrezeptor-Antagonisten, neuromuskulär blockierende Stoffe, Cholesterinesterase-Hemmstoffe, Adrenozeptor-Agonisten, indirekt wirkende Sympathomimetika, Methylxanthine, alpha-Adrenorezeptor-Antagonisten, Mutterkomalkaloide, beta-Adrenozeptor-Antagonisten, Inaktivierungshemmstoffe, Antisympathonika, 5-HT-Rezeptor-agonisten, Histaminrezeptor-Agonisten, Hiastaminrezeptor-Antagonisten, Analgetika, Lokalanästhetika, Sedativa, Antikonvulsiva, Konvulsiva, Muskelrelaxantien, Antiparkinsonmittel, Neuroleptika, Antidepressiva, Lithium, Tranquillantien, Immunsuppressiva, Antirheumatika, Antiarrhythmika, Antibiotika, ACE-Hemmer, Aldosteronrezeptor-antagonisten, Diuretika, Vasodilatatoren, positiv inotrope Substanzen, antithrombotische/thrombolytische Substanzen, Laxantien, Antidiarrhoioka, Pharmaka bei Adipositas, Uricostatika, Uricosurika, Lipidsenker, Antidiabetika, Anithypoglykämia, Hormone, Iodsalze, Threostatika, Eisen, Vitamine, Spurenelemente, Virostatika, Antimykotika, Antituberkulotika und Substanzen zur Tumorchemotherapie. Bevorzugt bezieht sich das Merkmal auf einen variablen Bestandteil des tierischen und/oder menschlichen Körpers. Viele dieser Analyten können zur Charakterisierung des Gesundheitszustandes von Individuen in einer Körperflüssigkeit, wie beispielsweise Lymphe, Galle, Urin, Tränenflüssigkeit, Speichel, Liquor, interstitielle Flüssigkeit und/oder insbesondere Blut, bestimmt werden, insbesondere bei chronischen Erkrankungen, wie z. B. Herzinsuffizienz, Niereninsuffizienz. Vorteilhafterweise dient das Sensorsystem zum Nachweis eines Mitglieds der Cystatin-Familie der Cysteinprotease-Inhibitoren und insbesondere zum Nachweis von Cystatin C und ist somit ein Cystatin-C-Sensor.

Des Weiteren soll unter einem Sensor insbesondere ein Bauteil verstanden werden, das eine optische, physikalische, chemische und/oder elektrochemische Eigenschaft des Merkmals in einer Umgebung des Sensors qualitativ und/oder quantitativ etwa als Messgröße erfassen kann. Ferner soll unter dem zweiten Merkmalsträger beispielsweise ein Analyt und unter dem ersten Merkmalsträger ein weiterer Analyt und insbesondere ein Gegenspieler und/oder ein Antagonist des Analyten verstanden werden. Das Merkmal und der zweite Merkmalsträger können auch dasselbe Objekt oder Molekül sein, insbesondere wenn das Merkmal durch die An- oder Abwesenheit des zweiten Merkmalsträgers/Analyten bestimmt wird. Ferner ist der zweite Merkmalsträger bzw. der Analyt zumindest zum Zeitpunkt des Nachweises am Sensor vorhanden, kann aber auch zu anderen Zeitpunkten, beispielsweise im Ruhezustand des Sensorsystems, vorhanden sein. Der Merkmalsträgerrezeptor kann beispielsweise allgemein von Peptiden, Antigenen, Aptameren, molekular-geprägten Polymeren und Polynukleotiden mit n ≥ 1 Monomereinheiten (Ribonukleinsäure - RNA, Desoxyribonukleinsäure - DNA, Peptid-Nukleinsäure - PNA, Locked-Nukleinsäuren - LNA) oder im speziellen von einem Rezeptor, einem Kanal, einem Transportprotein und/oder insbesondere von einem Antikörper oder einem Teil eines Antikörpers gebildet sein.

Vorteilhafterweise ermittelt der Sensor zumindest eine elektrische Zustandsgröße. Hierbei soll unter einer elektrischen Zustandsgröße beispielsweise ein Widerstand, ein Strom, und insbesondere eine Spannung verstanden werden. Durch dieses Messprinzip kann ein Sensorsystem mit geringem Platzbedarf und Energieverbrauch zur Verfügung gestellt werden. Zudem wird vorgeschlagen, dass der Sensor zumindest eine Spannungsänderung ermittelt. Diese Spannungsänderung wird durch einen Ladungsunterschied des ersten Merkmalsträgers gegenüber dem zweiten Merkmalsträger hervorgerufen, wodurch der Sensor einen ladungssensitiven Sensor darstellt. Mittels dieser Art der Bestimmung kann ein sehr sensitives System bereitgestellt werden.

Ferner ist es vorteilhaft, dass der Sensor zumindest ein Halbleiterbauelement aufweist. Hierbei soll unter einem Halbleiterbauelement ein FET (Feldeffekttransistor)-basiertes aktives Bauelement wie beispielweise ein seFET ("extended gate" Feldeffekttransistor), ein ISFET (ionensensitiver Feldeffekttransistor), ein EPROM (electrically erasable programmable readonly memory) oder ein EEPROM (electrically erasable programmable readonly memory), ein Kondensator, ein Nanoröhrchen, ein Nanodraht und/oder jedes andere, vom Fachmann für einsetzbar erachtetes Halbleiterbauelement verstanden werden. Alternativ kann auch ein impedimetrisches System als Sensor verwendet werden. Durch diese Ausgestaltung kann das Sensorsystem besonders einfach in einem miniaturisierten Format realisiert werden.

Des Weiteren wird vorgeschlagen, dass zumindest ein Bereich des Sensors mit zumindest dem Merkmalsträgerrezeptor beschichtet ist. Dieser Bereich wird bevorzugt von einem messrelevanten Bereich und/oder einer aktiven Sensoroberfläche gebildet und weist besonders bevorzugt ein so genanntes "floatendes gate" auf und/oder der Bereich wird von diesem gebildet. Ferner ist der Bereich mit einer Vielzahl von Merkmalsträgerrezeptoren beschichtet, eine Dichte hängt hierbei von Charakteristika des verwendeten Trios an erstem und zweitem Merkmalsträger sowie dem Merkmalsträgerrezeptor ab und wird vom Fachmann nach seinen Fachkenntnissen entsprechend ausgesucht. Zudem kann der Merkmalsträgerrezeptor mittels eines Linkers, wie beispielsweise Polyethylenglycol, Protein A, Protein G, an der Oberfläche gebunden sein. Linker können in diesem Zusammenhang auch kleinere organische Moleküle, z.B. EDC, einzelne Aminosäuren oder in der Regel kurze Polypeptide der Länge n=6-12 (N= Anzahl verbundener Aminosäuren) sein. Dadurch kann die Ladungsübertragung von dem an dem Merkmalsträgerrezeptor gebundenen ersten Merkmalsträger konstruktiv einfach auf das Halbleiterbauelement bzw. dessen "gate" übertragen werden.

Ein vielseitig einsetzbares Sensorsystem kann erreicht werden, wenn es einen Verdrängungsassay umfasst. Hierbei ist der Merkmalsträgerrezeptor von einem Molekül gebildet, das eine Antigenerkennungsstelle aufweist, also ein Antigenbindungspartner, wie beispielsweise ein polyklonaler oder monoklonaler Antikörper, ist. Hierdurch kann konstruktiv einfach eine spezifische und selektive Wechselwirkung mit dem Antigen erreicht werden. Hierfür wird vorgeschlagen, dass der zweite Merkmalsträger von einem ein Antigen aufweisenden Analyten gebildet ist. Zudem ist der erste Merkmalsträger von einem Antagonist des Analyten gebildet, der ebenso das Antigen aufweist. Im Folgenden werden die Begriffe zweiter Merkmalsträger und Analyt und erster Merkmalsträger und Antagonist synonym verwendet. Der Antagonist ist ein artifiziell hergestelltes oder modifiziertes insbesondere rekombinantes Molekül, das die gleiche molekulare Antikörper-Erkennungsstelle wie der Analyt besitzt. Diese Erkennungsstelle kann beispielsweise mittels "molecular engineering" und eines so genannten "Epitop-Mapping" mit folgender rekombinante Genklonierung und Proteinexpression ermittelt werden. Hierbei wird anhand der Aminosäuresequenzen des Antikörpers und des Antigens eine genaue Bestimmung einer molekularen Antikörper-Antigen Interaktionsstelle durchgeführt. Zudem weist der erste Merkmalsträger bzw. der Antagonist eine höhere Ladung wie der zweite Merkmalsträger bzw. der Analyt auf. Diese hohe Ladung wird dadurch erreicht, dass an den modifizierten Antagonist Moleküle mit hoher Ladung gekoppelt werden. Diese Moleküle sind beispielsweise anionische oder kationische Polymere, wie zum Beispiel hetero- oder homo-Polypeptide. Bevorzugt einsetzbar wäre hier beispielsweise Poly-L-Lysin. Durch diese Modifikation erfährt der erste Merkmalsträger nicht nur eine hohe Ladung, sondern auch eine großes Volumen, wodurch er vorteilhaft in einem Sensorinneren zurück gehalten werden kann. Da man den Antagonisten relativ frei rekombinant/bioinformatisch designen kann, kann ein hoher Signal-Rausch Abstand bei Verdrängung, ohne Modifikation des eigentlichen Analyten, realisiert werden, wodurch eine empfindliche Messung ermöglicht wird. Die hohe Ladungsdichte des oben genannten Antagonisten kann zum Beispiel durch rekombinante Klonierung erreicht werden. Beispielsweise kann für den Nachweis von Cystatin C einer geeigneten Grundsequenz rekombinant ein weiterer Proteinanteil zugefügt werden, der die benötigte Antikörperbindungsstelle nicht modifiziert und der vorzugsweise aus einer Aminosäure besteht, die eine Ladung trägt. Geeignete Aminosäuren, die eine Ladung tragen sind Lysin, Arginin und Histidin, sowie Aspartat (als die ionisierte Form von Asparaginsäure) und Glutamat als die ionisierte Form von Glutaminsäure, die beide bei pH-Werten über 5 negativ geladen sind.

Das Sensorprinzip beruht auf einem markierungsfreien immunologischen Nachweisverfahren in dem ein körpereigenes Biomarkermolekül konzentrationsabhängig und reversibel gemessen wird. Das Sensorsystem arbeitet somit mit einer besonders empfindlichen Methode basierend auf einem immunologischen Verdrängungsassay zum Nachweis bzw. zur Konzentrationsbestimmung des Analyten in Verbindung mit seinem Antagonisten und deren Antigenbindungspartner. Die Sensoroberfläche weist die gebundenen und selektiv den Analyten erkennende Merkmalsträgerrezeptoren auf. Bei Abwesenheit des Analyten sättigen die im Sensorinneren vorhandenen Antagonisten durch Bindung der Merkmalsträgerrezeptoren die aktive Sensoroberfläche. Die hohe Ladung des Antagonisten erzeugt auf der sensitiven Oberfläche des Halbleiterbauelements einen messbaren Ladungsübertrag, wodurch ein zuverlässiges, driftfreies und bei Bedarf doch korrigierbares Messsignal auf dem seFET generiert wird. Durch die Sättigung des Messsensors mit dem Antagonisten beträgt das Messsignal bei Abwesenheit des Analyten 100%.

Ist nun der Analyt an der aktiven Oberfläche vorhanden, konkurrieren der Antagonist und der Analyt um die Antigenerkennungsstelle des Antigenbindungspartners. So kommt es zu einer reversiblen Verdrängung des mit einer hohen Ladung versehenen Antagonisten, der an den Antigenbindungspartner gebunden ist, durch das eigentliche Antigen des Analyten. Es bildet sich ein konzentrationsabhängiges Gleichgewicht zwischen gebundenen Analyten und gebundenem Antagonisten, wobei der Ladungsübertrag für Analyt und Antagonist unterschiedlich ist. Insgesamt kann ein umso geringeres Messsignal abgeleitet werden, je mehr Analyt gebunden ist. Durch den großen Ladungsunterschied von Analyt und Antagonist ist die Änderung der Konzentration deutlich detektierbar, das gewünschte Prinzip der Messungsverstärkung wird angewendet. Die Analytkonzentration ist proportional zum gemessenen Signal. Sinkt die Konzentration an Analyten im Blut und somit auch im Sensorinneren, binden wieder vornehmlich Antagonisten an den Merkmalsträgerrezeptor und das Messsignal am seFET steigt wieder an.

Da die gesamte Bindungsenergie durch die erkennende Sequenz im Antigen bereitgestellt wird, spielt eine Masseänderung zwischen zwei sich gegenseitig verdrängenden Molekülen bei ausreichendem Abstand der Moleküle zueinander keine entscheidende Rolle in der Messung. Durch die Generierung des Referenzsignals im Sensor selbst (bei Abwesenheit des Antigens) wird auch ein Referenzpunkt der Messung bereitgestellt, um Sensordrift oder allmähliche Sensordegradation (durch Autolyse der Moleküle, Degradation der Messsensorschicht im seFET oder Einflüsse des Körpers auf das Implantat) herausrechnen zu können.

Durch die Sättigung des Messsensors mit dem Antagonisten, bei Abwesenheit des Analyten, ist gegebenenfalls eine interne Driftkorrektur des Messsensors nötig, da sich bei unvermeidlicher allmählicher Sensordegradierung das maximal mögliche Messsignal auch ohne Anwesenheit des Analyten allmählich erniedrigt. In der Elektronik des Messsensors kann diese allmähliche konzentrationsunabhängige Signalabnahme in Form einer erfahrungsbasierten Driftkorrektur erfasst und eliminiert werden.

Die Probleme, dass die meisten Analyten (z.B. Proteine) nur geringe, schwer nachweisbare Ladungen tragen und die bisherigen ladungssensitiven Nachweismethoden nicht sensitiv genug für solch kleine Moleküle mit geringen Ladungen waren, kann mit einem Verdrängungsassay und einem Antagonisten, der hohe Ladungen trägt, umgangen werden. Durch die Verdrängung des Antagonisten mit hoher Ladung durch den Analyten mit einer geringen eigenen Ladung, kann die Änderung der Analytkonzentration sehr deutlich messbar gemacht werden, da es durch die hohe Ladung des Antagonisten im analytfreien Zustand zu einer Signalverstärkung kommt. Zudem wird durch den Verdrängungsassay die Sensitivität des seFETs erhöht, da sich durch die Verdrängung der hoch geladenen Antagonisten durch den Analyten das Messsignal sehr stark ändert. Dies führt ferner zu einer Verbesserung des Signal-Rausch Abstandes. Des Weiteren werden Störeffekte kleiner geladener Moleküle und Stoffe in der Messsubstanz verringert, da eventuelle unspezifische Interaktionen nur einen sehr geringen Ladungsunterschied im Vergleich zur Verdrängung des hoch geladenen Antagonisten hervorrufen. Auch kann ein solches Sensorsystem Energie- und Platz sparend ausgestaltet werden, wodurch es sich besonders gut für den *in vivo* Einsatz und eine Überwachung eignet.

Das Messverfahren muss vollständig und in kurzer Zeit reversibel sein, damit der Sensor "wiederverwendbar" und über einen längeren Zeitraum hinweg einsetzbar ist. Hierzu muss der Merkmalsträgerrezeptor bzw. der Antikörper mit hoher Spezifität aber geringerer Affinität zum Antigen als herkömmliche Antikörper ausgestattet werden. Er muss zudem abhängig von der Konzentration des Analyten im Messmedium den Analyten und den Antagonisten im Gleichgewicht binden. Es ist bekannt, dass Antikörperfragmente (künstlich modifizierte, verkleinerte Antikörper) die gleiche Spezifität aber eine geringere Affinität zu einem Antigen besitzen als unmodifizierte Antikörper und somit in der Sensorik einfacher eingesetzt werden können (vgl. Saerens et al., "Antibody Fragments as Probing in Biosensor Development", Sensors 2008, 8, 4669-4686). Es ist weiterhin bekannt, dass Antikörperfragmente im Vergleich zu vollständigen Antikörpern viel seltener menschliche Immunreaktionen hervorrufen, da besonders die für die Bindung unrelevanten aber immunogenen konstanten Regionen gerne deletiert werden.

Der Merkmalträgerrezeptor kann beispielsweise ein Teil eines polyklonalen oder monoklonalen Antikörpers, wie ein Fab-, ein scFv-, ein hsFv-, ein dsFV-, ein ds-scFc-Fragment, eine VH- oder eine VL-Domäne sein (für eine Zusammenfassung der möglichen einsetzbaren Antikörperfragment, -domänen und -modifikationen siehe Saerens et al.). Eine Antigen-Antikörper-Bindung sollte hier relativ schwach sein und eine Affinität des Antikörpers bzw. des Antikörperfragments sollte sich etwa im µ-molaren Bereich befinden. Eine Bindungskonstante der Antikörperbindung soll hierbei vorteilhaft unter 1x 10⁻⁹ mol/l, bevorzugt unter 5x 10⁻⁸ mol/l, besonders bevorzugt bei etwa 1x 10⁻⁸ mol/l liegen. Auf die Bindungsstärke des Antikörpers bzw. Antikörperfragments kann sowohl durch Selektion während der Isolierung, als auch während der rekombinanten Optimierung Einfluss genommen werden. Die hierzu verwendeten Methoden sind Standardmethoden der Molekularbiologie (z.B. Phage-Display, Peptid-Display). Der finale Merkmalsträgerrezeptor ist artifiziell. Durch die geringere Affinität der Antikörperfragmente zu dem Analyten und dem Antagonisten (im Vergleich zu herkömmlichen Antikörpern) ist die Bindung zwischen den Bindungspartnern reversibel, wodurch messbare Gleichgewichtszustände schnell aufgebaut und eingestellt werden können. Dadurch können Waschschritte im Antikörperassay vermieden werden, was eine Miniaturisierung des Sensorsystems und eine Implantation erlaubt.

Zudem sollen die Antikörper bzw. Antikörperfragmente langzeitstabil sein - gegenüber klassischen Antikörper-Nachweisverfahren muss eine deutlich höhere Langzeitstabilität gewährleistet sein. Somit wird in einer weiteren Ausgestaltung der Erfindung vorgeschlagen, dass der Merkmalträgerrezeptor und/oder der erste Merkmalsträger chemisch, biologisch und/oder genetisch modifiziert oder "gehärtet" sind. Hierbei handelt es sich insbesondere um Bereiche der Moleküle, die für die Bindung des Antigens und des Antagonisten unwichtig sind. Ferner darf sich eine eingebrachte chemische Modifikation nicht auf die Bindung auswirken. Für eine gute Langzeitstabilität müssen Antikörperfragmente bereitgestellt werden, die beispielsweise gegenüber Proteasen und chemischer Degradation stabil sind (vgl. Saerens2008 et al.).

Möglichkeiten der Modifikation sind beispielsweise:
1. Entfernung nicht benötigter Bestandteile des Proteins (genetische Modifikation)
2. Entfernung von bekannten Proteaseschnittstellen im Protein (genetische Modifikation), wie z.B. das Entfernen oder Austauschen der Aminosäure Serin oder anderer einzelner Aminosäuren in bestimmten Aminosäuresequenzabfolgen was den Angriff und die Degradation des Proteins durch bspw. Serinproteasen verhindert
3. Einbau "unphysiologischer" Aminosäuren in den Antikörper, welche nicht im "Wirtssystem" angegriffen werden können (Expression des Proteins in heterologen Systemen)
4. Spezielle sekundäre Modifikationen der Antikörperoberfläche, die vom Wirtssystem nicht angegriffen werden kann (alternative Glykosilierung), wie z.B. Methylierungen, die das Angreifen bestimmter Proteasen oder die spontane Degradation von Aminosäuren verhindern können,
5. Chemische Modifikation (z.B. gerichtete Oxidation), die einen Abbau unter physiologischen Bedingungen erschwert oder behindert.

Die möglichen Modifikationen hängen auch von den Anforderungen an den Antikörper bzw. das Antikörperfragment sowie an den Antagonisten ab. Es sind nicht alle Methoden gleichzeitig nötig, sondern die Möglichkeiten verstehen sich eher als ein Baukastenprinzip. Gute Ergebnisse lassen sich in der Regel jedoch bereits einfach durch Möglichkeit 1 und 2 erzielen. Durch die Modifikationen der Antikörper bzw. des Antikörperfragments sowie des Antagonisten können diese im Gegensatz zu üblichen Biomolekülen langzeitstabil ausgeführt werden und über einen längeren Zeitraum im Sensor aktiv sein sowie Änderungen der Analytkonzentration verfolgen und nachweisen.

Des Weiteren kann es vorteilhaft sein, wenn das Sensorsystem zumindest eine organische Membran aufweist, mittels der zumindest ein Reservoir des Sensors abschließbar ist. In diesem Zusammenhang soll unter dem Begriff "organische Membran" eine Trennschicht und/oder ein dünner Film verstanden werden, die/der zumindest einen Bestandteil aufweist, der auf einer Kohlenstoffverbindung basiert. Die organische Membran enthält bevorzugt einen polymeren Stoff und/oder ist von einem polymeren Stoff gebildet, wobei sich dieser chemisch so herstellen lässt, dass eine Porengröße von Poren der Membran an die verwendeten Moleküle und das Messprinzip angepasst ist. Die Membran ist bevorzugt als semipermeable Membran ausgebildet. Der Term "abschließbar" soll hier nicht bedeuten, dass ein Stofftransport zwischen dem Reservoir und einem Außenregion völlig unterbunden ist, er sagt lediglich aus, dass ein Raum definiert wird, in dem bestimmte Bestandteile des Sensorsystems angeordnet sind bzw. zurückgehalten werden. Unter einem "Reservoirs eines Sensors" bzw. einem "Sensorreservoirs" soll hier ein Raum, eine Kammer und/oder eine Kavität des Sensors verstanden werden, mit dem das Nachweissystem des Sensors in Kontakt steht und/oder an und bevorzugt in dem das Nachweissystem bzw. der Verdrängungsassay angeordnet ist. Ein der semipermeablen Membran gegenüberliegender Boden des Reservoirs weist bevorzugt das "Gate" des Halbleiterbauelements bzw. des seFET auf. Ferner schließt das Sensorreservoir zumindest ein Volumen ein und insbesondere ein Probenvolumen, das das nachzuweisende Merkmal enthalten bzw. aufweisen kann. Durch die organische Membran kann vorteilhaft festgelegt werden, welche Moleküle mit dem Sensor in Kontakt kommen können und welche nicht. Die Membran und der Sensor sind so mit dem Gehäuse des Sensors verbunden, dass ein Stoffaustausch nur über die Porenmembran und nicht über die Verbindungsstelle zwischen Porenmembran und Gehäuse möglich ist.

Hierbei ist die organische Membran vorteilhaft so ausgebildet, dass der erste Merkmalsträger bzw. der Antagonist zu jeder Zeit im Reservoir zurückhaltbar ist. Somit kann ein Verlust an einem Bestanteils des Assays konstruktiv einfach verhindert werden. Zusätzliche werden negative Einflüsse des freigesetzten Antagonisten im Körper des Patienten vermieden. Ferner ist die organische Membran so ausgebildet, dass sie zumindest zeitweise für den zweiten Merkmalsträger bzw. den Analyten passierbar ist. Durch die semipermeable Membran, die den Analyten in das Sensorinnere filtriert, den Ladungsträgerantagonisten aber nicht hinauslässt, wird ein wieder verwendbarer, langzeitstabiler Sensor ermöglicht, wodurch eine Miniaturisierung und somit eine Implantation möglich gemacht wird. Zudem wird verhindert, dass Moleküle wie bspw. anderen Blutbestandteilen, die größer als eine Porengröße der Membran sind, in das Reservoir eindringen können und so Störeffekte ausgeschaltet werden können. Dadurch kann ein Signal-Rausch-Verhältnis (Signal-to-noise ratio) realisiert werden, dass so niedrig ist, dass ein ausreichend hohes, messbares Signal detektierbar ist. Ferner können somit Waschritte eliminiert werden, die sonst zu einer unzumutbaren Sensor- bzw. Implantatgröße geführt hätte.

Es wird zudem vorgeschlagen, dass die organische Membran steuerbar ausgeführt ist. In diesem Zusammenhang soll unter "steuerbar" verstanden werden, dass die Membran mittels zumindest eines Signals von zumindest einem ausgewählten Anfangszustand in zumindest einen ausgewählten Endzustand überführt werden kann. Vorteilhaft ist das Signal ein von außerhalb des Sensorsystems einwirkender Einfluss, wie beispielsweise Strahlung, Infrarot, Ultraschall, ein elektrisches Feld, ein magnetisches Feld, sichtbares Licht, ein Protein, ein Peptid, ein Polyelektrolyt, eine pH-Wertänderung, eine Veränderung der Ionenkonzentration, eine Temperaturänderung und/oder jede andere, dem Fachmann für sinnvoll erscheinende Einwirkung. Bevorzugt ist ein Volumen der Membran steuerbar.

Des Weiteren wird vorgeschlagen, dass die steuerbare, organische Membran zwischen einem geöffneten und einem geschlossenen Zustand des Reservoirs stufenlos oder in Stufen veränderbar ist. Unter "stufenlos" soll hier die Möglichkeit verstanden werden, die Öffnungsweite der Membran bis zu einer maximalen Grenze kontinuierlich oder beliebig weit einzustellen. Die Einstellung ist bevorzugt "reversibel" also umkehrbar. Durch die Realisierung der Änderung kann das sich im Sensorreservoir befindliche Nachweissystem vorteilhaft vor interferierenden Molekülen geschützt werden, die den Sensor angreifen, degradieren und/oder zerstören könnten. Auch Störfaktoren, die eine einwandfreie Funktion des Sensors beinträchtigen können, werden so vorteilhaft minimiert. Dadurch kann ein besonders langlebiges Sensorsystem bereitgestellt werden. Eine geeignete steuerbare, organische Membran ist beispielsweise in DE 10 2008 010 876 A1 beschrieben.

Ferner ist es zweckmäßig, wenn die steuerbare, organische Membran vor einem ersten Einsatz bzw. einem ersten Messbetrieb des Sensors geschlossen ist, wodurch der Sensor vor seiner ersten Inbetriebnahme effektiv vor störenden Einflüssen, wie Schmutz, Staub, zu hohe Feuchtigkeit, Trockenheit, Temperaturschwankungen und/oder schädlichen Molekülen geschützt ist. Des Weiteren kann es vorteilhaft sein, wenn die steuerbare, organische Membran zwischen den einzelnen Messungen verschließbar ist, wodurch das Sensorsystem Komponenten stabil realisiert werden kann.

Zudem ist vorgesehen, dass die organische Membran zumindest eine Pore aufweist, deren Durchmesser reversibel veränderbar ist, wodurch ein Zustand der Membran und ein Übertritt des Analyten in das Probevolumen konstruktiv besonders einfach flexibel, insbesondere für unterschiedliche Moleküle und/oder Analyten, gestaltet werden kann. Ferner sind zumindest ein Öffnen und/oder ein Schließen der Pore der Membran steuerbar. Die Pore ist bevorzugt eine Nanopore und ein Porendurchmesser ist dabei abhängig vom nachzuweisenden Analyten. Bevorzugt wird der Porendurchmesser so gewählt, dass die Membran für Moleküle des Analyten durchlässig ist, jedoch für größere Moleküle eine Barriere darstellt. Grundsätzlich können die Poren, falls es sich beim nachzuweisenden Analyten um ein Protein oder ein ähnlich großen Analyten handelt, einen maximalen Durchmesser von 1 µm, bevorzugt von maximal 250 nm, weiterhin bevorzugt von maximal 100 nm, vorteilhafterweise von maximal 50 nm und besonders bevorzugt von maximal 10 nm aufweisen. Bei kleineren Analyten können die Poren einen maximalen Durchmesser von 500 nm, bevorzugt von maximal 100 nm, weiterhin bevorzugt von maximal 50 nm, vorteilhafterweise von maximal 10 nm und besonders bevorzugt von maximal 1 nm aufweisen. Generell kann die Pore auch eine beliebig andere Kontur, wie eine dreieckige, rechteckige, sternförmige, ovale und/oder jede andere, dem Fachmann für zweckdienlich erscheinende Kontur aufweisen, wobei die maximale Erstreckung die oben genannte Staffelung aufweist. Durch die Nanopore kann einfach verhindert werden, dass Strukturen, wie beispielsweise Zellen, große Moleküle oder Molekülaggregate, die eine größere Erstreckung wie der Durchmesser der Nanopore haben, aus der Sensorumgebung in das Probenvolumen eindringen, um dort das Nachweissystem zu stören. Vorteilhaft weist die Membran eine Vielzahl von gleichartigen Poren auf, die gleichmäßig über eine Oberfläche der Membran verteilt angeordnet sind. Grundsätzlich wäre auch eine inhomogene Verteilung der Poren denkbar. Vorteilhafterweise ist der Porendurchmesser stufenlos oder in Stufen einstellbar, was eine Anwendung mit einer Vielzahl an Analyten ermöglicht.

Vorteilhaft weist die steuerbare, organische Membran zumindest ein Material auf, dessen Redoxzustand änderbar ist. Hierbei kann der Redoxzustand chemisch, elektrisch und/oder auf jeder anderen, dem Fachmann für anwendbar erscheinende Weise änderbar sein. Vorteilhafterweise kann die steuerbare, organische Membran in Abhängigkeit von dem Auslösesignal ihren Zustand so ändern, dass zumindest ein Teil der Membran durchlässig für den Analyten ist, wodurch der Analyt einen guten Zugang zum Nachweissystem erhält.

Besonders bevorzugt ist die organische Membran elektrisch steuerbar ausgeführt. Hierbei ist insbesondere der Porendurchmesser der Pore bzw. der Poren der Membran elektrisch steuerbar. Dies erfolgt vorteilhafterweise durch Anlegen einer Spannung von maximal ca. 2 V. Bei vollständig geöffneten Nanoporen hat das seinen Redoxzustand ändern könnende Material sein minimales Volumen. Das Anlegen einer Spannung führt zu einer Volumenvergrößerung des Materials, wodurch sich der freie Durchmesser verkleinert. Die Volumenvergrößerung ist entweder abhängig von der Höhe der Spannung, wenn diese eine bestimmte konstante Zeitspanne anliegt, oder sie ist von der Zeitspanne abhängig, während derer eine konstante Spannung anliegt. In beiden Fällen muss die Spannung nur für eine bestimmte Zeitdauer angelegt werden. Die Reversion des Vorganges erfolgt analog durch Anlegen einer Spannung umgekehrter Polarität, welche dann wieder zu einer Volumenreduktion des Materials führt. Die Volumenänderung ist ebenfalls von dem Strukturdesign des Materials abhängig.

Bevorzugt ist das Material, das seinen Redoxzustand ändern kann, ein elektroaktives Material bzw. ein elektroreaktives Polymer. Wird als elektroaktives Polymer z. B. eine Mischung aus Polypyrrol (PPy) und Dodecylbenzolsulfonsäure (DBS) eingesetzt, werden während einer spannungsgesteuerten Reduktion des Polymers Natriumionen in das Polymer eingelagert. Diese Einlagerung von Natriumionen erzeugt eine stark laterale Volumenänderung des elektroaktiven Polymers, das damit die Poren für den Analyten verschließt. Die Reversibilität dieses Vorganges ermöglicht eine kontrollierte Öffnung und ein kontrolliertes Schließen der Poren und damit eine kontrollierte, wiederholbare Messung mittels des Sensors. Über den Grad der Reduktion des Polymers ist eine partielle Volumenänderung des Polymers möglich. Die Redoxzustände des elektroaktiven Polymers werden über unterschiedliche angelegte Spannungen erzeugt und bei einer Abschaltung der Spannung beibehalten. Dadurch kann das Polymer und damit der Porendurchmesser vorteilhaft auf verschieden große Analyten eingestellt werden. Als Alternative zu Polypyrrol (PPy) im oben beschriebenen elektroaktiven Polymer kann eine der folgenden Substanzen eingesetzt werden: PEDOT (poly(3,4-ethylenedioxythiophene), poly(3,4-dioxypyrrole) (PXDOP), Polyacetylen, Polyanilin, Polythiophen, Poly(phenylvinlen) und Derivate dieser.

In einer weiteren Ausgestaltung der Erfindung ist vorgesehen, dass die Membran bzw. das Redoxzustand verändernde Material bzw. das Polymer auf eine nanoporöse Substanz als Trägerstruktur aufgebracht ist, wodurch eine besonders gute Funktionalität erreicht werden kann. Bevorzugt ist die Membran bzw. das Redoxzustand verändernde Material bzw. das Polymer zumindest an einer Innenoberfläche von Poren der nanoporösen Substanz bzw. Trägerstruktur angeordnet. Hierdurch können die Nanoporen der Trägerstruktur vorteilhaft als Grundgerüst der Porenstruktur verwendet werden. Der Porendurchmesser ist dabei abhängig vom nachzuweisenden Analyten und angepasst an die oben genannten Durchmesser der Poren der Membran.

Die nanoporöse Substanz weist bevorzugt ein Metalloxid wie Al₂O₃, In₂O₃, MgO, ZnO, CeO₂ Co₃O₄ auf und/oder weist die Trägerstruktur für die steuerbare, organische Membran bzw. das Polymer besonders bevorzugt zumindest TiO₂ auf. Grundsätzlich wäre jedoch auch jede andere nanoporöse Substanz einsetzbar. Mittels der Verwendung von TiO₂ kann eine Trägerstruktur vorteilhaft eingesetzt werden, die besonders gewichtsarm, biokompatibel und bioinert ist. Zudem ist die Nanoporenstruktur, aufgebaut aus Nanoröhrchen, sehr einfach und reproduzierbar zu synthetisieren. Diese sehr regelmäßigen Strukturen können relativ einfach durch einen Anodisierungsprozess erzeugt werden. Die Porengröße und Schichtdicke dieses Substrats können durch Parameter während der Erzeugung leicht eingestellt werden. In der Regel ist die Schichtdicke mit mehreren hundert Mikrometern deutlich größer als der Durchmesser der Nanoröhrchen.

Bevorzugt ist das komplette Sensorsystem biologisch abbaubar, wodurch es nach seinem Funktionsverlust Patienten schonend nicht wieder aus dem Körper entfernt werden muss. Zudem kann verhindert werden, dass schädliche Stoffe, wie beispielsweise bei einem Einsatz eines nicht biokompatiblen und abbaubaren Sensors gesundheitsgefährdend in den Körper gelangen.

Der Sensor ist vorteilhaft zur quantitativen Bestimmung des Merkmals ausgebildet, wobei so eine Konzentrationsbestimmung des Analyten, beispielsweise in Körperflüssigkeiten, wie Blut, Urin, interstitieller Flüssigkeit oder Tränenflüssigkeit, einfach bestimmt werden kann. Besonders Bauteil und Baurraum sparend kann die quantitative Bestimmung erfolgen, wenn derselbe Bereich als ein erster Sensor und als ein zweiter Sensor vorgesehen ist. Hierbei ist der erste Sensor bevorzugt als ein Messsensor und der zweite Sensor als ein Referenzsensor ausgebildet. Der Messsensor und der Referenzsensor sind einstückig miteinander ausgebildet, wobei hier unter "einstückig" insbesondere verstanden werden soll, dass Messsensor und der Referenzsensor von demselben Bauteil gebildet sind und/oder nur unter Funktionsverlust voneinander getrennt werden können. Das Sensorreservoir ist so ausgebildet, das es in einem ersten Modus zur Referenzmessung und in einem zweiten, zeitlich dem ersten Modus nachfolgenden Modus zur Analytmessung dient. Hierbei wird eine Porengröße bei der Referenzmessung so eingestellt, dass sie kleiner als ein Durchmesser des Analyten ist, so dass dieser nicht in das Probevolumen eindringen kann. Moleküle oder Strukturen, die kleiner als der Analyt sind, können hingegen in das Probenvolumen eindringen. Bei der Analytmessung wird die Porengröße nun an die Größe des Analyten angepasst, wodurch dieser ins Probenvolumen eindringen kann. Das Messsignal der Referenzmessung wird nun als Hintergrundsignal von dem Messsignal der Analytmessung abgezogen, wodurch ein Endmesswert ermittelt wird. So kann das Hintergrundsignal von Störeffekten konstruktiv einfach bestimmt werden.

Bei der alternativen Ausgestaltung des Sensorsystems ist vorgesehen, dass das Sensorsystem zumindest einen zweiten Sensor, der als Referenzsensor ausgebildet ist, aufweist. Somit sind zwei unterschiedliche Bereiche als ein erster Sensor und als ein zweiter Sensor vorgesehen, wodurch zeitsparend die Analyt- und Referenzmessung zeitgleich oder nacheinander erfolgen kann. Der erste und der zweite Sensor bzw. der Messsensor und der Referenzsensor sind bevorzugt räumlich voneinander getrennt im Sensorsystem angeordnet. Der zweite Sensor kann von jedem beliebigen dem Fachmann für anwendbar erscheinenden Sensor gebildet sein. Bevorzugt weist der zweite Sensor ebenso ein Referenzreservoir, das ein Referenzvolumen einschließt. Die beiden Sensoren unterscheiden sich bevorzugt durch ihre Porengröße. Die Porengröße der organischen Membran des ersten Sensors/Messsensors lässt den Analyten in das Probenvolumen. Eine kleiner eingestellte Porengröße der organischen Membran des zweiten Sensors/Referenzsensor hält den Analyten ab, in das Referenzvolumen zu gelangen. Durch Korrigieren des gemessenen Signals des Messsensors mittels des Referenzsignals erhält man den Endmesswert. Bevorzugt weisen die beiden Sensoren schaltbare, organische Membranen und insbesondere elektrisch schaltbare Membran auf, die sich durch eine Konfiguration ihrer schaltbaren Membranen und insbesondere durch die daran jeweils eingestellte Porengröße unterscheiden. Die unterschiedlichen Porositäten für Mess- und Referenzsensor werden durch entsprechend unterschiedlich hohe Spannungen und/oder Zeitdauer der anliegenden Spannung an den zu den Sensoren gehörenden Membranen eingestellt.

Dieser Aufbau hat den Vorteil, dass sowohl Messsensor und Referenzsensor das gleiche Nachweissystem enthalten und somit ein Drift und Alterungsprozesse gleich ablaufen. Somit können bei Ermittlung des Endmesswerts mittels des Messsensors, des Referenzsensors oder beider Sensoren Aussagen über den Drift (z.B. Degradierung des Nachweissystems, Veränderung der Temperatur und andere Effekte) des Sensorsystems vorteilhaft gesammelt werden. Über einen geeigneten Korrekturterm können die nachfolgenden Messwerte bzgl. der Drift korrigiert werden, bzw. über eine geeignete Methode (elektrischer, mechanischer oder chemisch/biochemischer Natur) kompensiert werden. Ist die Drift zu groß, dass diese Mechanismen nicht mehr greifen, kann bei einem Arraysystem gleicher Sensorsysteme ein neues Sensorsystem aktiviert werden.

Beim Betreiben des erfindungsgemäßen medizinischen Sensorsystems mit der steuerbaren, organischen Membran wird diese reversibel zwischen einem geöffneten und einem geschlossenen Zustand des Reservoirs verändert. Hierbei kann zum Nachweis des Merkmals in einem ersten Schritt ein erster Durchmesser einer Pore der steuerbaren, organischen Membran eingestellt und in einem zweiten Schritt ein zweiter Durchmesser der Pore eingestellt werden, wobei der erste Durchmesser kleiner als der zweite Durchmesser ist. Alternativ kann zum Nachweis des Merkmals an einer steuerbaren, organischen Membran eines zweiten Sensors ein erster Durchmesser einer Pore der steuerbaren, organischen Membran eingestellt und an der steuerbaren, organischen Membran des ersten Sensors ein zweiter Durchmesser einer Pore der steuerbaren, organischen Membran eingestellt werden, wobei der erste Durchmesser kleiner als der zweite Durchmesser ist. Hierdurch kann das Messergebnis der Messung mit Analyten einfach um das Messergebnis des Hintergrundsignals korrigiert werden.

Es ist zu erwarten, dass mit dem Nachweissystem interferierende und dieses störende Moleküle, wie andere Ladungsträger, ebenfalls in die sensitive Region des Sensors eindringen bzw. sich an das "Gate" des FETs anlagern können. Diese können sich an die sensitive Sensoroberfläche anlagern und bilden einen unspezifischen Ladungsübertrag, der direkte Auswirkungen auf ein Messsignal zeigt. Dadurch kann es sowohl zu einer starken Drift wie auch zu einer völligen Störung des Messsignals kommen, so dass kein Signal mehr abgeleitet werden kann. Um dies zu minimieren, wird in einer vorteilhaften Ausgestaltung vorgeschlagen, dass der Sensor zumindest eine Passivierungsschicht aufweist, welche auf zumindest einem Bereich des Sensors aufgebracht ist und dazu ausgebildet ist Bindungsstellen, insbesondere unspezifische Bindungsstellen abzusättigen. Dieser Bereich stellt bevorzugt denselben Bereich dar, wie der mit Merkmalsträgerrezeptor bzw. mit Antigenbindungspartner beschichtete Bereich und insbesondere das "gate" des FETs. Alternativ oder zusätzlich kann auch eine Oberfläche, die direkt mit einer Implantationsstelle und/oder einer Körperflüssigkeit in Kontakt kommt, insbesondere eine Außenfläche eines das Sensorsystem aufweisende Implantats, gegen die Anlagerung von Störfaktoren aus dem Körper, insbesondere aus der Körperflüssigkeit passiviert werden.

Zur Realisierung der Passivierung können chemisch inerte, langzeitstabile, insbesondere hydrophile Substanzen und/oder Polymere wie z.B. Polyethylenglycol (PEG), PEG-Derivate, Hydrogele, Dextran und/oder jede andere, vom Fachmann für einsetzbar erachtete Substanz eingesetzt werden. Diese Substanz kann insbesondere physikalisch adhärieren und/oder kovalent an die Oberfläche gebunden werden. Grundsätzlich könnte die Anbindung auch nach jeder anderen, vom Fachmann als sinnvoll erachtete Art erfolgen. Eine Kettenlänge und -struktur des Polymers hängen von der Art des eingesetzten Merkmalsträgerrezeptors ab. Ob der Merkmalsträgerrezeptor vor oder nach der Aufbringung der Passivierungsschicht bzw. des passivierenden Polymers auf die Oberfläche gebracht wird, hängt von der Art des eingesetzten Polymers und des Merkmalsträgerrezeptor-Linkers bzw. dessen Bindungsstellen ab. Bei PEG bietet sich beispielsweise an, zunächst die Merkmalsträgerrezeptor -Linker auf die Oberfläche zu binden, deren Anbindungsstellen für den Merkmalsträgerrezeptor zu schützen, dann das PEG aufzubringen und in einem letzten Schritt die Merkmalsträgerrezeptor anzubinden. Wichtig ist, dass die Passivierungsschicht sehr dünn ist um die Ladungsverschiebung mit hoher Sensitivität zu messen. Daher sind Materialien mit kleiner Dielektrizitätskonstante und kleine Schichtdicken im Nanometerbereich bevorzugt. Eine Möglichkeit dünne Schichten für die Passivierung und für die Unterdrückung von unerwünschten Messsignalen zu gestalten mit der gleichzeitigen Möglichkeit Ankergruppen für Linkermoleküle bereit zu stellen bieten Plasmapolymere. Diese können konform in einem Postprozessingschritt auf das Bauelement, insbesondere einem der FET, aufgebracht werden.

Zur Realisierung dieser Nanofilme können unterschiedliche Monomere herangezogen werden. Als bewährte Ausführungsform einer biokompatiblen und proteinabweisenden Nanoschicht dienen einfache Kohlenwasserstoffe wie CH₄ oder C₂H₂. Diese werden in einer Hochfrequenzentladung vorzugsweise mittels magnetron-unterstützten Niederfrequenzentladung abgeschieden. Die Dielektrizitätskonstante kann zwischen 2 und 2,5 und die Schichtdicke zwischen 10nm und 70 nm variiert werden wobei eine Schichtdicke von 20nm bevorzugt ist.

An diese Schichten können mittels Benzophenon-Reaktionen auch Linkermoleküle angekoppelt werden.

Als hydrophobe Beschichtung können auch Fluorkohlenwasserstoffe als Monomere verwendet werden wie C₂F₄ oder C₄F₈ und in der Gasentladung polymerisiert werden. Hier ist die Dielektrizitätszahl höher und kann zwischen 3 und 3,5 eingestellt werden.

Mittels der Passivierungsschicht werden Interferenzen mit der Oberfläche des FETs - hervorgerufen insbesondere durch geladene Moleküle - unterbunden. Dies führt vorteilhaft zu einem geringen Störmesssignal. Durch das Verdrängungsprinzip mit großem Antagonist, der nicht durch die semipermeable Membran aus dem Sensor entweichen kann und dem Gleichgewichtsmechanismus zwischen Merkmalsträgerrezeptor, Analyt und Antagonist, sowie der geeigneten Passivierungsschicht auf der sensitiven Oberfläche für ein konstantes Signal-Rausch Verhältnis wird das Problem der in immunologischen Nachweisreaktionen sonst obligaten Waschschritte umgangen.

Sensoren, die *in vivo* implantiert sind, haben insbesondere bei Blutkontakt große Probleme mit der Langzeitstabilität. Typischerweise zeigen sie nach dem Einbringen in den Körper eine unspezifische Proteinadsorption. Diese adsorbierten Proteine verlieren ihre Tertiär- bzw. Quartärstruktur wenigstens teilweise und dienen Zellen als Ankersubstrate für eine Anlagerung. Hierdurch ausgelöst bilden sich eine undefinierte Zellbedeckung und/oder eine extrazelluläre Matrix aus Protein-Fasern (z. B. Kollagen) auf der Oberfläche des Sensors oder des Implantats, wie beispielsweise der semipermeablen Membran, aus. Dieser Vorgang wird allgemein als Biofouling bezeichnet. Außerdem kann es kann zu einer Thrombenbildung an der Oberfläche des Sensors oder des Implantats kommen.

Ferner bedeuten diese Ablagerungen, dass sich für den Analyten eine zeitlich nicht stabile Diffusionsbarriere aufbauen kann, die direkte Auswirkungen auf das Messsignal zeigt. Durch die Zellbedeckung bzw. die extrazelluläre Matrix kann es sowohl zu einer Drift wie auch zu einer Verzögerung (Delay) des Messsignals kommen, so dass der Sensor nur mit einer zeitlichen Verzögerung äußere Veränderungen des Merkmals oder der Analytkonzentration detektieren kann. Die Diffusionsbarriere kann so groß werden, dass die Analyten nicht mehr zum Sensor gelangen und hier kein Signal mehr abgeleitet werden kann.

Um dem entgegen zu wirken wird vorgeschlagen, dass das Sensorsystem zumindest eine Oberflächenbeschichtung als Anti-Foulingbeschichtung, insbesondere als eine Anti-Biofoulingbeschichtung, aufweist, welche auf zumindest einem Bereich des Sensors aufgebracht ist und dazu ausgebildet ist Wechselwirkungen mit Störstoffen zu verhindern. Dieser Bereich ist bevorzugt die organische semipermeable Membran, insbesondere deren Flächen, die mit Körperflüssigkeit in Kontakt kommen können. Störstoffe sind hier Bestandteile von Strukturen, wie Gewebe und/oder Körperflüssigkeiten, die mit dem Sensor und/oder dem Implantat in Kontakt kommen können und insbesondere Zellen und/oder molekulare Bestandteile, wie Proteine, Salze, Ionen und/oder jeder andere, vom Fachmann für schädlich erachtete Störstoff. Die Oberflächenbeschichtung weist z.B. hydrophile Polymere oder eine Hydrogelschicht (PEG und PEG-Derivate), die ein Anheften von Zellen und Molekülen unterbinden, auf. Mittels der Oberflächenbeschichtung als Anti-Foulingbeschichtung kann das Sensorsystem vorteilhaft und konstruktiv einfach störunanfällig gestaltet werden.

Gleichzeitig oder alternativ kann eine Sensor- und/oder Implantatoberfläche so modifiziert werden, dass die Reaktion des Körpers in eine für die langzeitstabile Funktion des Sensors sinnvolle Richtung gelenkt wird. Beispielsweise kann eine entsprechende Beschichtung, eine Mikro- und/oder eine Nanostrukturierung, auf/in die Sensor- und/oder Implantatoberfläche auf- bzw. eingebracht werden, welche beispielsweise das Einwachsverhalten des eingebrachten Implantats und/oder die Reaktion des Körpers auf dieses positiv beeinflussen. Es können zu Strukturierung auf der Oberfläche sowohl runde sphärische, sphärische, zylindrische, kegelförmige, quadratische, rechteckige, wie auch längliche Strukturen aufgetragen wie auch abgetragen werden. Dazu zählen Rillen, Röhren, Vollzylinder, Kugeln, Halbkugeln, Quader und Würfel.

Ferner können beispielsweise auf die Sensor- und/oder Implantatoberfläche gezielt Strukturen aus den Körperflüssigkeiten, wie z.B. bestimmte Proteine oder Zellen angelockt, immobilisiert und die Zellen zu Proliferation gebracht werden. Dadurch kann das Einwachsverhalten und das Biofouling positiv beeinflusst werden, zur Ausbildung einer nach einer Einwachsphase zeitlich unveränderlichen Diffusionsbarriere. Zudem wirkt eine so modifizierte Oberfläche nicht thrombogen wodurch ein Gesundheitsrisiko für den Patienten verringert wird.

Es wird erfindungsgemäß vorgeschlagen, dass das Sensorsystem zumindest einen Bereich aufweist, auf dessen Oberfläche spezifische Erkennungsmarker für Zellen angeordnet sind, wobei die Erkennungsmarker ausgewählt sind aus der Gruppe bestehend aus Peptiden, Proteinen, Antikörpern, Antigenen, Aptameren, molekular-geprägten Polymeren und Polynukleotiden mit n ≥ 1 Monomereinheiten (Ribonukleinsäure - RNA, Desoxyribonukleinsäure - DNA, Peptid-Nukleinsäure - PNA, Locked-Nukleinsäuren - LNA). Im Sinne der vorliegenden Erfindung bedeutet "molekular geprägt" die Bereitstellung eines Polymergerüstes mit Erkennungsdomänen. Durch molekulares Prägen sind mit Informationen versehene Polymere zugänglich. Beispielsweise können Polymere durch radikalische Polymerisation in Gegenwart eines Templates (das sich im günstigsten Fall durch Waschschritte wieder entfernen lässt) mit der nötigen Selektivität versehen werden, um zu ähnlichen Strukturen Affinitäten auszubilden. Ein solches Verfahren ist aus Vaidya et al., A. & L. Fischer. 2004: "Altering glucose oxidase to oxidase Dgalactose through crosslinking of imprinted protein" (ChemBioChem 5 (1): 132-135) bekannt.

Die Art des Epitops bestimmt dabei die Polymerauswahl. Geeignet sind Acrylate und Methacrylate als Grundgerüst. Azo-bis-(isobutyronitril) (AIBN) ist der bevorzugte Radikalstarter. Die Reaktion kann in Lösungsmitteln wie Dioxan, CHCl₃ oder THF, aber auch in einer anderen Substanz durchgeführt werden. Durch Copolymerisate mit Acylacetat (hydrophile Gruppen; können zur Erzeugung von OH-Gruppen gut verseift werden), Acylamin; Acylsäuren oder Styrol (Wechselwirkung mit aromatischen Epitopen) können neben hoher Selektivität auch hohe Bindungsaffinitäten erzeugt werden.

Ein "spezifischer Erkennungsmarker" im Sinne dieser Erfindung ist dabei ein Molekül, das spezifisch für die Anlagerung eines bestimmten Zelltypes ausgebildet ist. Mit anderen Worten, ein "Erkennungsmarker für Zellen" im Sinne dieser Erfindung ist eine Verbindung oder ein Teil einer Verbindung, der von einem, zwei oder drei Zelltypen (bevorzugt einem) spezifisch erkannt wird und eine Bindung der Zellen dieses Typus oder dieser Typen an einer Oberfläche, auf der sich der Erkennungsmarker befindet, bewirken kann. Zellen anderer Typen zeigen dagegen keine solche Reaktion. Im vorliegenden Fall wird bevorzugt die Migration und Proliferation von Endothelzellen (EC) gefördert.

Bevorzugt sind dabei allgemein die Zelltypen, die mit Hilfe von Transmembranproteinen (Integrinen) die Erkennungsmarker auf der Implantatsoberfläche erkennen, ausgewählt aus der Gruppe, der Zellen, die Integrine tragen. Besonders bevorzugt sind dabei Zellen, die zur alfaVBeta3 (αvβ₃) Unterfamilie gehören. Neben den schon genannten Endothelzellen besitzen auch Endothelvorläuferzellen die gewünschten Erkennungssequenzen. (Blind et al.: "A novel Drug-Eluting Stent coated with an Integrin-Binding Cyclic Arg-Gly-Asp Peptide Inhibits Neointimal Hyperplasia by Recruiting Endthelial Progenitor Cells"; JA College of Cardiology; Vol. 47, No9, 2006; Garcia A.J.: Get a Grip, integrins in cellbiomaterial interactions"; Biomaterials 26; 7525-7529, 2005). Die Verwendung von Peptidsequenzen auf Stents ist in WO 2008/143933 A1 beschrieben. Hier soll ebenfalls ein beschleunigtes Einheilen, durch Ausbildung eines Zellrasens, erreicht werden.

Das beschriebene Vorgehen ist für theranostische Anwendungen, also solche, die mindestens eine diagnostische, sensorische und/oder therapeutische Funktion haben, noch nicht bekannt. Bekannt und Stand der Technik sind Bemühungen die Gewebsbedeckung zu kontrollieren respektive wie durch PEG-ylierungen zu verhindern. Das Sensorsystem mit dem Sensor, kann Teile eines Implantats sein. Zur Vereinfachung wird im Folgenden von einem das implantierbarer theranostischer Artikel bzw. Artikel gesprochen, wodurch sowohl ein Sensor bzw. ein Sensorsystem alleine und ein Implantat mit Sensorsystem und Sensor verstanden werden soll.

Das Vorsehen bestimmter Erkennungsmarker an der Oberfläche eines implantierbaren theranostischen Artikels bewirkt bei der geeigneten Auswahl der Marker abhängig z. B. vom Einsatzort des implantierbaren theranostischen Artikels, dass die Artikeloberfläche im implantierten Zustand eine Grenzfläche präsentiert, auf die der Körper mit einer definierten und nach einem bestimmten Zeitraum sich nicht weiter verändernden dünnen Vernarbung bzw. Verkapselung reagiert. Überraschend ist in diesem Zusammenhang insbesondere, dass nach einer verhältnismäßig kurzen Anwachsphase ein im Prinzip sich nicht weiter veränderndes Gewebe auf dem implantierten theranostischen Artikel entsteht. Durch diese biomimetische Oberfläche wird der Artikel dann nicht mehr vom Körper als fremdes Objekt identifiziert.

Der Kern dieses Erfindungsaspekts stellt somit eine neue Herangehensweise dar. Während in der Vergangenheit verstärkt versucht wurde, bioinerte Oberflächen und Nanostrukturierungen für Implantatanwendungen zu schaffen, die mit dem Nachteil der Thrombogenität und mangelnden Spezifität verbunden waren, wird mit der vorliegenden Erfindung erreicht, dass durch die spezifischen Erkennungsmarker für Zellen eine (weitgehend) konstante und biomimetische Oberfläche entsteht. Hierbei ist der Umfang der Zellbedeckung auch durch die Konzentration der spezifischen Erkennungsmarker für Zellen auf der Oberfläche des implantierbaren theranostischen Artikels steuerbar. Im Prinzip entsteht aber eine verhältnismäßig geringe Zellbedeckung, die sich nach einer Anwachsphase (oder Einheilung) im Laufe der Zeit nicht weiter vergrößert. Dadurch stellt diese Schicht eine überraschend geringe Diffusionsbarriere dar, wobei ebenfalls überraschend ist, dass ein stationärer Zustand auch im Sinne einer Diffusion von Analyten erreicht wird.

Durch die geeignete Bindungsmethode ist es dem Fachmann möglich, dass faktische Einwachsverhalten des (gewünschten) Zelltyps zu steuern. Vorliegend ist insbesondere die Bildung einer Endothelzellschicht bevorzugt. Hierbei ist es, insbesondere bei einer Bewachung der Membran, wichtig, dass eine Diffusion des Analyten in das Reservoir des Sensorsystems bzw. das Sensorinnere ungehindert stattfinden kann. Die meisten interessanten Moleküle sind dabei klein genug, um ein gutes Diffusionsverhalten durch die Endothelzellschicht in das Sensorinnere zu gewährleisten.

Für Absorption geeignete Artikeloberflächen sind dabei bevorzugt ausgewählt aus der Gruppe bestehend aus Titan, med. Edelstahl wie bevorzugt 316L, CoCr, Magnesium und Polymeren. Polymere können dabei sowohl unter Einsatzbedingungen im Körper abbaubar als auch am Körper permanent sein. Für kovalente Bindungen geeignete Gruppen auf der Oberfläche des implantierbaren Artikels sind bevorzugt ausgewählt aus der Gruppe bestehend aus Hydroxyrest, Aminorest Carbonylrest und Mercaptogruppe.

Erfindungsgemäß bevorzugt ist, dass die Erkennungsmarker über Adsorption, kovalente Bindung oder Linker an den implantierbaren Artikel gebunden sind. Ein Verbindungs-Linker im Sinne der vorliegenden Erfindung ist ein Molekülteil, das die Verbindung zwischen dem spezifischen Erkennungsmarker für Zellen und der Oberfläche des implantierbaren theranostischen Artikels chemisch gewährleistet. Der Verbindungs-Linker ist aufgebaut aus einer Ankergruppe und einer Spacergruppe. Die Spacergruppe weist dabei eine Kettenlänge von 1-30 bevorzugt 5-12 Atomen auf. Geeignete bevorzugte Ankergruppen sind: Acylsäure, Phosphonate, Thiole, Isocyanate und besonders bevorzugt Isothiocyanate. Als Spacer finden bevorzugt Verwendung: PEG, Polyprolin, Adipinsäure, bevorzugt Aminohexansäure. Weitere Reagenzien zur Kopplung wie N,N'-Carbonyldiimidazol (CDI), 1-(3-Dimethylaminopropyl)-3-ethylcarbodiimid (EDC) oder Disulfosuccinimidyl-tatrat (DST) sind unter bestimmten Einsatzbedingungen ebenfalls bevorzugt.

Bevorzugt im Sinne der Erfindung ist, dass der spezifische Erkennungsmarker ein Oligopeptid ist. Oligopeptide umfassen bis zu zehn Aminosäuren und können sowohl aufgrund ihrer Größe als auch auf Basis ihrer Funktionsweise als Erkennungsmarker für bestimmte Zelltypen besonders gut im Sinne der vorliegenden Erfindung eingesetzt werden. Ganz besonders bevorzugt im Rahmen dieser Erfindung ist ein erfindungsgemäß implantierbarer Artikel, wobei der molekulare Erkennungsmarker eine RGD- oder cRGD-Sequenz umfasst oder aus ihr besteht.

Besonders bevorzugt sind im Rahmen der vorliegenden Erfindung ist der spezifische Erkennungsmarker für Zellen, ausgewählt aus der Gruppe bestehend aus Verbindungen der Formel 1 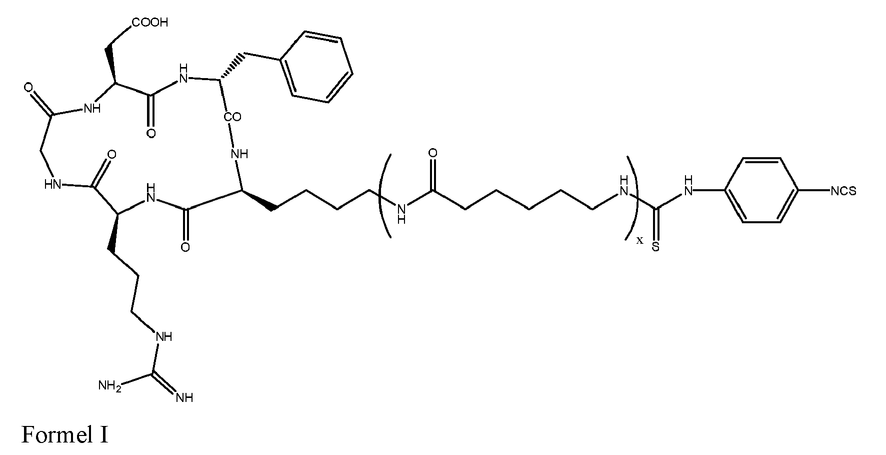 x = 0, 1, 2, 3, 4, 5 oder 6
und
Verbindungen der Formel II 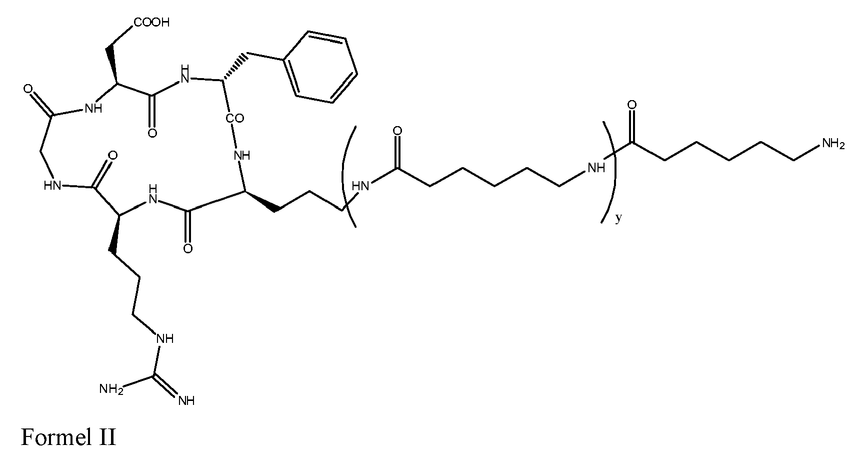 y = 0, 1, 2, 3, 4 oder 5.

Erfindungsgemäß bevorzugt ist ferner, dass die Oberfläche des Artikels bzw. Teile der Oberfläche metallischen, keramischen oder Polymercharakter besitzt. Darüber hinaus kann die Oberfläche des Implantats bzw. Teile der Oberfläche abhängig von der Anwendung hydrophobe oder hydrophile Eigenschaften besitzen, sie können sowohl einen kationischen oder anionischen bzw. metallischen Charakter tragen.

Erfindungsgemäß bevorzugt kann die Oberfläche des implantierbaren theranostischen Artikels weitere Modifikationen tragen, die spezielle Bestandteile der Körperflüssigkeit binden oder abweisen. Als solche Modifikationen können anorganische oder organische Moleküle über physikalische Absorption oder kovalente Bindung an die Artikeloberfläche geknüpft werden, wie z. B. Polymere, Peptide, Proteine, Aptamere, molekular-geprägte Polymere, RNA, DNA, PNA, LNA, siRNA und Nanopartikel. Es ist auch eine teilweise oder vollständig bioresorbierbare oder biodegradierbare Oberfläche denkbar.

Zweckmäßigerweise geht die Erfindung ferner aus von einem medizinischen Sensorarray mit zumindest zwei Sensorsystemen. Hierbei kann es sich um die gleichen Sensorsysteme handeln, die zeitlich nacheinander geschaltet den gleichen Analyten nachweisen bzw. dessen Konzentration bestimmen. Weiterhin können die Sensorsysteme gleichzeitig für die Analytmessung und für die Referenzmessung benutzt werden. Durch diese Ausgestaltung kann vorteilhaft eine beschränkte Lebensdauer eines Sensorsystems, mittels eines Arrays von mehreren Sensorsystemen auf eine gute Gesamtlebensdauer verlängert werden. Alternativ ist ebenfalls ein Array von Sensorsystemen möglich, die gleichzeitig oder zeitlich nacheinander verschiedene Analyten und/oder deren Konzentration nachweisen können. Für einen solchen Sensorarray bzw. einen Multisensor können mehrere spezifische Antikörper-Antigen-Antagonisten-Trios entwickelt werden.

Vorteilhaft geht die Erfindung zudem aus von einem medizinischen Implantat mit zumindest einem medizinischen Sensorsystem und/oder mit einem medizinischen Sensorarray. In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise ein Implantat zur Aufzeichnung von physiologischen Parametern, ein Herzschrittmacher, ein Defibrillator, ein Kardioverter, ein Hirnschrittmacher, ein Nervenstimulatoren, ein Nierenschrittmacher, ein Zwölffingerdarm-Schrittmacher, ein Herzimplantat, künstliche Herzklappen, ein Cochleaimplantat, ein Retina Implantat, ein zahnmedizinisches Implantat, ein Implantat zum Gelenkersatz, eine Gefäßprothesen oder besonders vorteilhaft ein Stent, wie ein Koronarstent, ein Nierenarterienstent oder ein Harnleiterstent sowie ein Drug-Delivery-System. Mit so einem Implantat können insbesondere Erkrankungen, wie beispielsweise Herzinsuffizienz, Bluthochdruck, Niereninsuffizienz und/oder Diabetes Mellitus als häufige, behandlungsintensive und somit teure chronische Erkrankungen besser beobachtet und therapiert werden. Für ein Implantat eignet sich die beschriebene Modifikation der Oberfläche beispielsweise der Membran besonders gut, da viele der Implantatoberflächen regelmäßig mit Körperflüssigkeit, insbesondere Blut, in Kontakt kommen, wo ein definiertes Einwachsen besonders hohe Bedeutung besitzt.

Besonders überraschend in diesem Zusammenhang ist - wie oben bereits angedeutet - dass für die erfindungsgemäßen implantierbaren theranostischen Artikel ein Sensor mit der erfindungsgemäß vorzusehenden Oberflächenbelegung durch spezifische Erkennungsmarker für Zellen erreicht werden kann, dass die entstehende Zellschicht in einem ausreichenden Maße durchlässig für die jeweiligen Analyten ist. Dies gilt sogar für den Fall, dass der Teil der Oberfläche des implantierbaren Sensors, der durch eine semipermeable Membran (durchlässig für den gewünschten Analyten) gebildet wird, ebenfalls an seiner (Außen-) Oberfläche spezifische Erkennungsmarker für Zellen im Sinne dieser Erfindung umfasst.

Ferner kann der Sensor bzw. das *in vivo* Messgerät eine Telemetrieeinheit enthalten, mittels derer die gemessenen Werte an ein externes Gerät zum Auswerten und/oder Anzeigen übertragen werden können. Die Telemetrieeinheit kann uni- oder bidirektional ausgeführt sein, so dass im zweiten Fall eine Steuerung des implantierten Sensors oder Messgerätes durch ein externes Gerät erfolgen kann. In Folge der Ausgabe des Messsignals kann eine manuelle Medikamenteneinnahme erfolgen. Es ist weiterhin möglich, dass das Implantat bzw. der Artikel ein Wirkstoffdosierungssystem enthält, wobei unter einem Wirkstoffdosierungssystem hier ein implantierbarer Artikel gemeint ist, der über einen längeren Zeitraum kontrolliert ein Medikament absondern kann. Ein solches Wirkstoffdosierungssystem kann einen "Closed Loop" darstellen, d.h. es kann, in Abhängigkeit von der ermittelten Analytkonzentration und getriggert durch den Sensor, ein automatisches Medikamentenabgabesystem gesteuert werden und zur Abwendung kritischer Ereignisse eine Medikamentenabgabe erfolgen. Es ist weiterhin möglich, dass vom externen Gerät Daten an das Wirkstoffdosierungssystem übermittelt werden, die als Trigger für die Substanzabgabe fungieren. Diese Datenübertragung kann automatisch erfolgen. Alternativ ist es möglicht, dass manuell ein Trigger für das Wirkstoffdosierungssystem gesetzt werden kann. Mittels der erfindungsgemäßen Ausgestaltung können kritische physiologische Werte zeitnah erfasst, ausgewertet, gegebenenfalls korrigiert oder deren Auftreten verhindert werden.

Ebenso kann der Sensor bzw. das *in vivo* Messgerät eine Teilkomponente eines Body-Area-Networks und/oder eines Tele-Monitoring-Systems sein, d.h. parallel erfassen weitere ebenfalls über drahtlose Telemeterie miteinander und/oder mit einem externen Gerät kommunizierende Sensoren physiologisch relevante Parameter wie Druck, Puls, EKG, EEG, biochemische Größen und/oder andere, dem Fachmann für zweckdienlich erscheinende Parameter. Der implantierbare, miniaturisierbare Sensor kann beispielsweise über einen Chip in einer Herzschrittmacherkapsel und über ein Elektrodenkabel steuerbar sein. Die Energieversorgung (Batterie, Akkumulator) liefert die für die Funktion des Sensors und die Datenübertragung die nötige Energie. Darüber hinaus ist es möglich, die nötige Energie mittels einer im Implantat befindlichen Brennstoffzelle zu liefern. Eine alternative Energieversorgung eines Implantats besteht darin, eine externe Energiequelle vorzusehen und die zum Betrieb des Implantates benötigte Energie mittels induktiver Kopplung zum Implantat zu übertragen.

Die Erfindung geht ferner aus von einem Verfahren zum Betreiben eines medizinischen Sensorsystems zum Nachweis von zumindest einem Merkmal eines tierischen und/oder menschlichen Körpers mit zumindest einem Sensor, einem ersten Merkmalsträger und einem Merkmalsträgerrezeptor, wobei sich der erste Merkmalsträger zumindest in einem Merkmalsparameter von einem zumindest zum Zeitpunkt des Nachweises vorhandenen zweiten Merkmalsträger unterscheidet.

Es wird vorgeschlagen, dass der zumindest eine Sensor bei dem Nachweis des zumindest einen Merkmals *in vivo* angeordnet wird und vom Sensor eine Merkmalsänderung ermittelt wird, welche durch eine reversible Verdrängung des zweiten Merkmalsträgers vom Merkmalsträgerrezeptor durch den ersten Merkmalsträger hervorgerufen wird. Es wird insbesondere ein Ladungswechsel bzw. eine Reduzierung der gebundenen Ladung an dem Sensor bzw. dem "gate" des FETs gemessen und ausgewertet. Dies wird durch die Verdrängung des hoch geladenen Antagonisten vom Antigenbindungspartner durch den nur geringe Ladung tragenden Analyten bewirkt. Durch dieses Messprinzip wird ein Verfahren bereitgestellt, dass *in vivo* über Monate und Jahre zuverlässig arbeiten kann. Ferner reagiert es sensitiv und schnell auf Bedingungsänderungen in der Messsubstanz.

Die Erfindung ist nachfolgend beispielhaft, anhand in Zeichnungen dargestellter Ausführungsbeispiele, näher erläutert. Es zeigen:
- Fig. 1: eine schematische Ansicht eines erfindungsgemäßen Sensorsystems von oben,
- Fig. 2: einen Schnitt entlang der Linie 11-11 durch das Sensorsystem der Fig. 1 mit einem Nachweissystem in einer schematischen Darstellung,
- Fig. 3: einen Schnitt durch das Sensorsystem der Fig. 1 mit einem Messsensor und einem Referenzsensor mit unterschiedlich großen Poren in einer schematischen Darstellung,
- Fig. 4: das Sensorsystem gemäß der Fig. 1 mit weiteren Komponenten,
- Fig. 5A: ein Implantat ausgestattet mit einem Sensorsystem gemäß der Fig. 1,
- Fig. 5B: ein alternatives Implantat ausgestattet mit einem Sensorarray mit vier Sensorsystem gemäß der Fig. 1,
- Fig. 6: eine schematische Darstellung eines molekularen Imprintens von Polymeren,
- Fig. 7A: einen Schnitt analog der Linie 11-11 der Fig. 1 durch ein alternatives Sensorsystem mit steuerbarer Membran mit geschlossenen Poren in einer schematischen Darstellung,
- Fig. 7B: das Sensorsystem der Fig. 6A in einem geöffneten Zustand der Poren für eine Referenzmessung,
- Fig.7C: das Sensorsystem der Fig. 6A in einem geöffneten Zustand der Poren für eine Analytmessung,
- Fig. 8A: eine Detaildarstellung einer Pore aus Fig. 6A im geschlossenen Zustand und
- Fig. 8B: eine Detaildarstellung einer Pore aus Fig. 6C im geöffneten Zustand.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verwiesen.

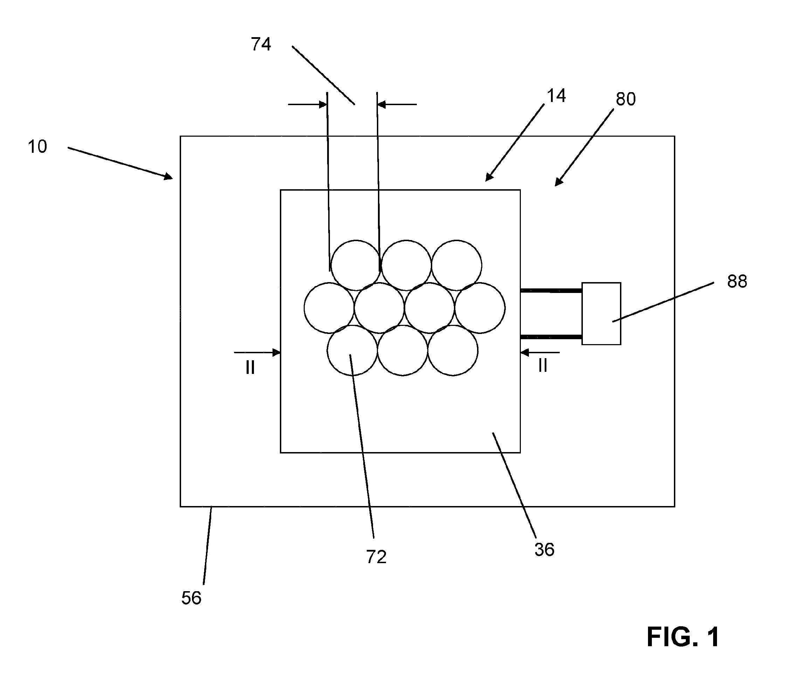

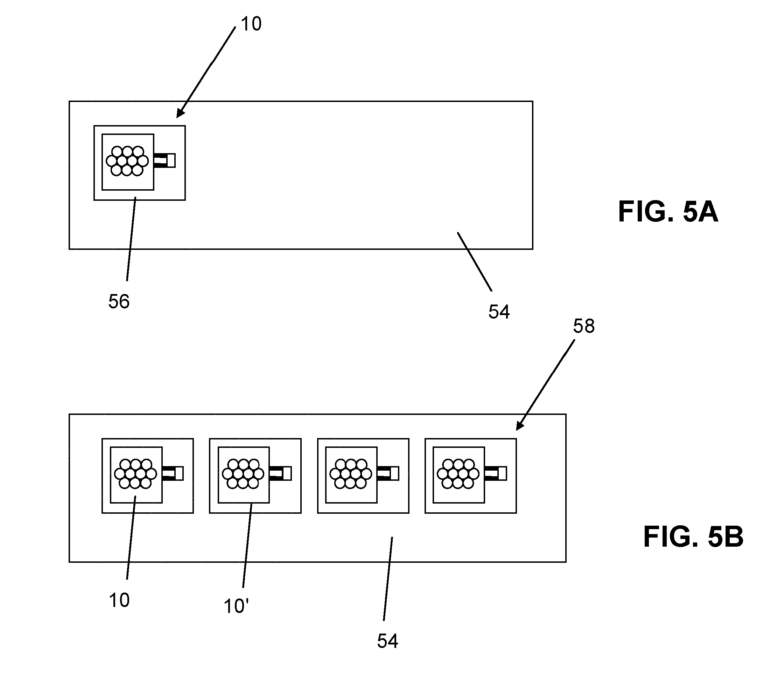

Die Fig. 1 zeigt ein medizinisches Sensorsystem 10 mit einem in einem Gehäuse 56 angeordneten Sensor 14 zum Nachweis von einem Merkmal 12 in einem hier nicht gezeigten menschlichen Körper schematisch von oben dargestellt. Mehrere Sensorsysteme 10, 10' können in einem medizinischen Sensorarray 58 zusammengefasst werden, wie dies in der Fig. 5B gezeigt ist Hierbei kann nach Verwendung eines ersten Sensorsystems 10 oder nach einem Erreichen dessen Ende einer Lebensdauer des ersten Sensorsystems 10 ein zweites Sensorsystem 10' aktiviert werden. Zudem ist in den Figuren 5A und 5B zu sehen, dass das Sensorsystem 10 (Fig. 5A) oder das Sensorarray 58 (Fig. 5B) in den menschlichen Körper implantiert werden kann, indem er/es mit einer nicht im Detail gezeigten Verankerungsvorrichtung an einem medizinischen Implantat 54 befestigt werden kann. Dieses Implantat 54 ist beispielsweise eine Formgedächtnisstruktur, wie ein Stent oder eine Mäanderstruktur zur Implantation in einer Arterie oder Vene (nicht gezeigt). Die Verankerung kann permanent sein oder wieder gelöst werden. Dadurch ist der Sensor 14 bei dem Nachweis des Merkmals *in vivo* angeordnet.

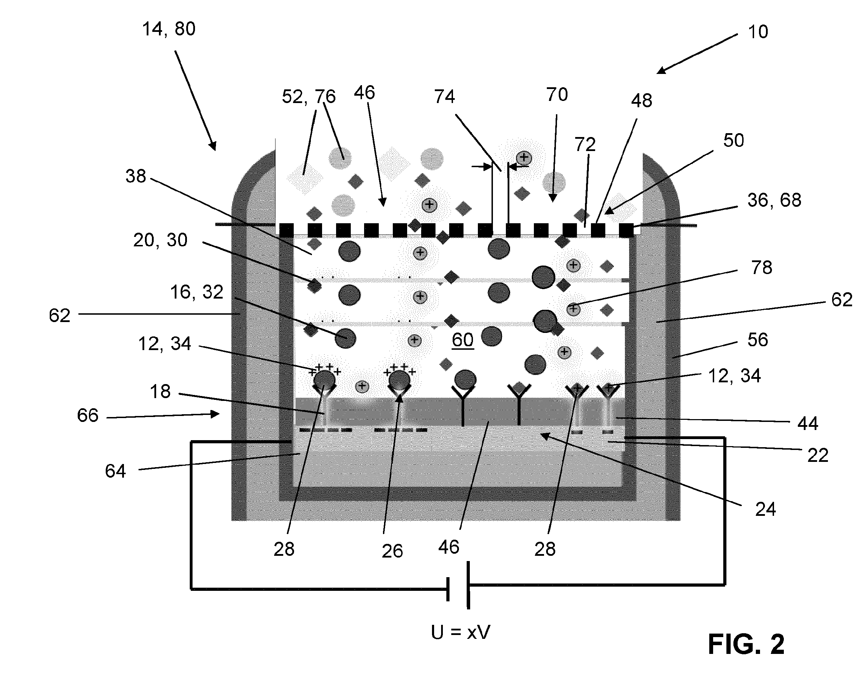

Wie in der Fig. 2, die einen Schnitt einlang der Linie 11-11 in Fig. 1 zeigt, zu sehen ist, weist der Sensor 14 ein Reservoirs 38 bzw. ein Sensorreservoir, auf, das ein Probenvolumen 60 an vier Seiten 62 und einem Boden 64 einschließt, wobei nur zwei Seiten 62 zu sehen sind. In dem Reservoir 38 bzw. am Boden 64 ist ein Nachweissystem 66 angeordnet. Zudem weist der Sensor 14 eine Selektionsstruktur 68 des Reservoirs 38 des Sensors 14 auf, die an einer sechsten, dem Boden 64 gegenüberliegenden Seite 70 des Reservoirs 38 angeordnet ist bzw. die sechste Seite 70 des Reservoirs 38 bildet. Die Selektionsstruktur 68 ist als eine organische semibermeable Membran 36 ausgebildet, mittels der das Reservoir 38 abschließbar ist.

Das Sensorsystem 10 bzw. das Nachweissystem 66 weist eine Vielzahl von Merkmalsträgerrezeptoren 18 auf, mit denen ein Bereich 24 des Sensors 14 bzw. des Bodens 64 mit einer dem Fachmann bekannten Dichte als Rezeptorschicht beschichtet ist. Der Boden 64 weist ein Halbleiterbauelement 22 in der Form eines extended-gate Feldeffektransistors (seFET) auf und der Bereich 24 stellt ein "Gate" des seFETs dar. Ein Merkmalsträgerrezeptor 18 ist von einem Molekül gebildet, das eine Antigenerkennungsstelle 26 aufweist und ist ein Fab-Fragment eines monoklonalen Antikörpers gegen ein nachzuweisendes Protein bzw. einen Analyten 30, der das Antigen 28 enthält. Der Merkmalsträgerrezeptor 18 wurde zudem mittels molekularbiologischer Verfahren verändert, hierbei wurde die Antigenerkennungsstelle 26 so modifiziert, dass eine Affinität für das Antigen herabgesetzt wurde, damit die Bindung reversibel erfolgen kann (nicht im Detail gezeigt). Eine Bindungskonstante der Antigen-Antigenerkennungsstelle 26, 28 beträgt etwa 1x 10⁻⁸ mol/l.

Der Analyt 30, in diesem Fall Cystatin C, stellt zudem einen zweiten Merkmalsträger 20 dar. Das Antigen 28 wird ferner von einem im Sensor 14 bzw. dessen Probenvolumen 60 vorliegenden Antagonist 32 des Analyten 30 präsentiert. Dieser Antagonist 32 ist ein artifizielles und rekombinant mit Epitop-Mapping entwickeltes Protein, welches eine Poly-L-Lysin-Modifikation trägt. Des Weiteren stellt er einen erste Merkmalsträger 16 als Bestandteil des Sensorsystems 10 bzw. des Sensors 14 dar. Ferner wurden der Antagonist 32 und der Merkmalsträgerrezeptor 18 molekularbiologisch so modifiziert, dass einige in deren Sequenzen enthaltenen, für die Anbindung des jeweiligen Gegenspielers unwichtigen Aminosäuren, die jedoch von stoffwechseleigenen Enzymen als Degradationsstartpunkt erkannt werden (z.B. Serin von Serinproteasen), durch andere, die Proteinstruktur nicht verändernde Aminosäuren, ersetzt werden. So werden die Moleküle vor enzymatischer Degradation im Körper schützen, wodurch die Langzeitstabilität erhöht wird (nicht im Detail gezeigt).

Die organische Membran 36 ist so ausgebildet ist, dass der erste Merkmalsträger 16 bzw. der Antagonist 32 zu jeder Zeit im Reservoir 38 zurückgehalten wird und dass der zweiten Merkmalsträger 20 bzw. der Antagonist diese passieren kann. Hierfür weist die aus einem Polymer gebildete organische Membran 36 mehrere Poren 72 auf, die homogen über die Oberfläche verteilt angeordnet sind. In der Fig. 1 sind der Übersichtlichkeit halber nur einige Poren 72 gezeigt. Zudem sind die Poren allen Figuren nicht maßstabsgetreu sondern vergrößert gezeigt. Ein Durchmesser 74 der Pore 72 der organischen Membran 36 muss für ein Durchdiffundieren des Analyten 30, Cystatin C größer als etwa 5 nm sein. Sie muss aber kleiner als 20 nm sein, damit der Antagonist 32 im Reservoir 38 zurückgehalten wird. Wäre der Analyt beispielsweise Glucose, würde ein Porendurchmesser von ca. 1 nm ausreichen, damit die Glucose die Membran passieren kann. Weitere Zellen oder Moleküle 76, die größer als der Porendurchmesser 74 sind werden von der Membran 36 zurückgehalten. Kleinstmoleküle 78 können die Membran 36 jedoch passieren. Die Membran 36 und das Halbleiterbauelement 22 sind so mit dem Gehäuse 56 verbunden, dass ein Stoffaustausch nur über die Poren 72 der Membran 36 und nicht über eine Verbindungsstelle zwischen der Membran 36 und dem Gehäuse 56 möglich ist.

Der erste und der zweite Merkmalsträger 16, 20 weisen beide das nachzuweisende Merkmal 12 auf. Hierbei stellt das Merkmal 12 eine Ladung 34 des ersten Merkmalsträgers 16 bzw. des Antagonisten 32 und des zweiten Merkmalsträgers 20 bzw. des Analyten 30 dar. Der erste Merkmalsträger 16 weist durch die poly-L-Lysin-Modifikation eine große positive Ladung 34 und eine höhere Ladung 34 wie der zweite Merkmalsträger 20 auf. Somit unterscheidet sich der erste Merkmalsträger 16 in einem Merkmalsparameter, nämlich der Ladungsintensität, von dem zweiten Merkmalsträger 20.

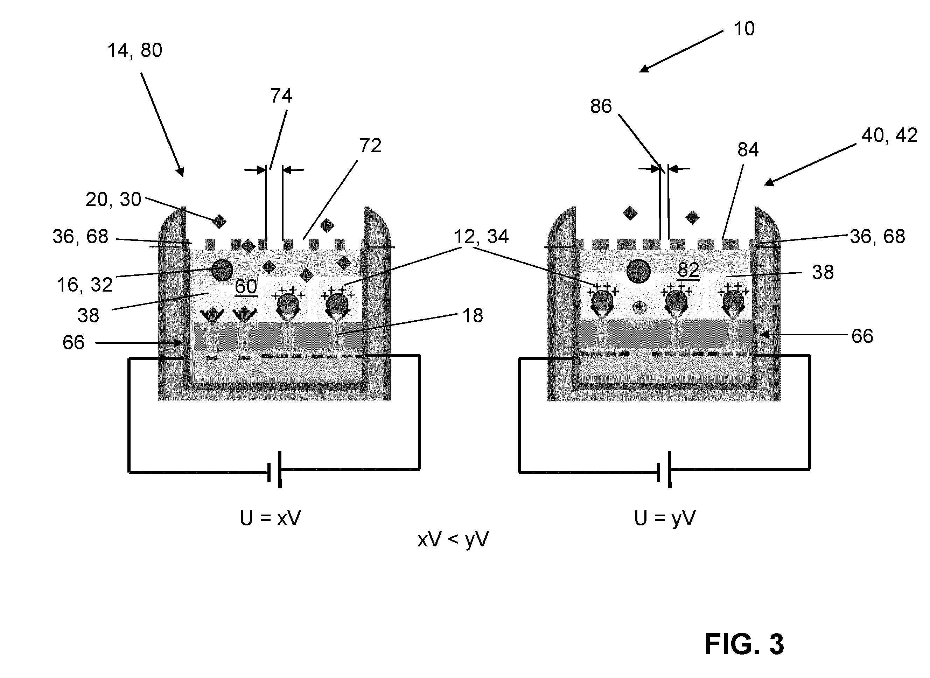

Das Sensorprinzip beruht auf einem markierungsfreien immunologischen Nachweisverfahren in dem der Analyt 30 konzentrationsabhängig und reversibel gemessen werden kann Das "Gate" des seFETs weist die gebundenen und selektiv den Analyten 30 erkennenden Merkmalsträgerrezeptoren 18 auf. Diese sind bei Abwesenheit des Analyten 30 und insbesondere auch vor der ersten Messung vom im Probenvolumen 60 vorhandenen Antagonisten 32 gesättigt (vgl. auch Fig. 3 rechts). Die hohe Ladung 34 des Antagonisten 32 erzeugt auf der sensitiven Oberfläche des Halbleiterbauelements 22 einen messbaren Ladungsübertrag, wodurch ein Messsignal auf dem seFET generiert wird. Durch die Sättigung des Sensors 14 mit dem Antagonisten 32 beträgt das Messsignal bei Abwesenheit des Analyten 30 100%.

Tritt nun der Analyt 30 aus der Messsubstanz, die den Sensor 14 umgibt und beispielsweise Blut ist, in das Probenvolumen 60 des Sensors über die semipermeable Membran 36 ein, kann dieser die vorhandene Bindung zwischen Antagonist 32 und Merkmalsträgerrezeptor 18 stören. Liegt nun der Analyt 30 an der aktiven Oberfläche vor, konkurrieren der Analyt 30 und der Antagonist 32 wegen ihres jeweils gleich ausgeführten Antigens 28 gleichberechtigt und gleich stark um die Antigenerkennungsstelle 26 des Antikörperfragments. Hierdurch kommt es durch das Antigen 28 des Analyten 30 zu einer reversiblen Verdrängung einiger der mit hoher Ladung 34 versehener und an den Merkmalsträgerrezeptor 18 gebundener Antagonisten 32. Es stellt sich ein konzentrationsabhängiges Gleichgewicht zwischen gebundenen Analyten 30 und gebundenem Antagonisten 32 ein, wobei der Ladungsübertrag für Analyt 30 und Antagonist 32 unterschiedlich ist. Insgesamt kann ein umso geringeres Messsignal abgeleitet werden, je mehr Analyt 30 gebunden ist.

Der Sensor 14 ermittelt elektrische Zustandsgröße bzw. eine Spannungsänderung. Durch den großen Ladungsunterschied von Analyt 30 und Antagonist 32 ist die Änderung der Konzentration deutlich detektierbar. Hierbei ist die Analytkonzentration proportional zum gemessenen Signal. Es wird also im Nachweisprozess vom Sensor 14 eine Merkmalsänderung bzw. Ladungsänderung ermittelt, welche durch die reversible Verdrängung des ersten Merkmalsträgers 16 vom Merkmalsträgerrezeptor 18 durch den zweiten Merkmalsträger 20 hervorgerufen wird. Sinkt die Konzentration an Analyten 30 im Blut und somit auch im Sensorinneren, binden wieder vornehmlich Antagonisten 32 an den Merkmalsträgerrezeptor 18 und das Messsignal am seFET steigt wieder an. In Fig. 2 ist auch zu sehen, dass Analyten 30 und Antagonisten 32, die die Antigenerkennungsstelle 26 nicht exakt gebunden haben, keinen Beitrag zur Generierung des Messsignals leisten.

Die auf das "Gate" des seFETs aufgebrachte Rezeptorschicht besetzt nicht alle Bindevalenzen der Oberfläche des "Gates". Weitere noch vorhandene freie bzw. ungesättigte Bindungsstellen 46 des Bereichs 24 des seFETs müssen gesättigt werden, so dass Interferenzen durch andere geladene Moleküle, wie Kleinstmoleküle 78, wirksam verhindert werden können. Demnach ist auf den Bereich 24 des seFETs eine Passivierungsschicht 44 aufgebracht, die dazu ausgebildet ist unspezifische Bindungsstellen 46 abzusättigen. Die Passivierungsschicht 44 ist von einem Polymer, wie z.B. Polyethylenglycol (PEG), gebildet. Zudem weist das Sensorsystem 10 eine Oberflächenbeschichtung 48 auf, welche auf einem Bereich 50 des Sensors 14 aufgebracht ist und dazu ausgebildet ist, Wechselwirkungen mit Störstoffen 52, wie Zellen und Bestandteilen oder Molekülen 76 der Messsubstanz, zu verhindern. Der Bereich 50 ist gebildet von einer Oberfläche der Membran 36, die mit der Messsubstanz in Berührung kommen kann. Grundsätzlich können auch andere Bereiche des Sensorsystems beschichtet sein.

Wie in Fig. 3 dargestellt, weist das Sensorsystem 10 zusätzlich zu dem als Messsensor 80 ausgebildeten Sensor 14 einen zweiten Sensor 40 auf, der als Referenzsensor 42 ausgebildet ist. Der Referenzsensor 42 führt eine Referenzmessung durch, bei der so viele Störsignale wie möglichst ermittelt werden sollen. Diese Sensoren 14, 40 sind räumlich voneinander getrennt im gleichen Gehäuse 56 angeordnet. Ferner weist jeder Sensor 14, 40 ein Reservoir 38 auf, das ein Probenvolumen 60 bzw. ein Referenzvolumen 82 einschließt. Jedes Reservoir 38 ist mit einer semipermeablen organischen Membran 36 ausgestattet. Die Membran 36 des Referenzsensors 42 weist Poren 84 auf, deren Durchmesser 86 kleiner ist als der Durchmesser 74 der Poren 72 der Membran 36 des Messsensors 80. Der Durchmesser 86 der Membran 36 des Referenzsensors 42 ist gerade so eingestellt, dass der Analyt 30 nicht in das Referenzvolumen 82 eindringen kann und beträgt beispielsweise ca. 5 nm. Alle kleineren Moleküle 78, die eine Bestimmung des Analyten 30 beeinträchtigen könnten, können hingegen eindringen. Die Referenzmessung ermittelt somit das 100% Signal bei Sättigung der Merkmalsträgerrezeptoren 18 mit dem Antagonisten 32 und den Signalen der restlichen mit dem "Gate" des seFETs interagierenden geladener Stoffe bzw. Kleinstmoleküle 78. Wie oben beschrieben, sind die Poren 72 der Membran 36 des Messsensors 80 mit ca. 10 nm so dimensioniert, dass der Analyt 30 bzw. das Cystatin C in das Probenvolumen 60 zu dessen Messung eindiffundieren kann. Zum Erhalten eines Endmessergebnisses der Konzentration an Analyt 30 wird nun das Messergebnis der Analytmessung um das Messergebnis der Referenzmessung korrigiert. Alternativ funktioniert das Messprinzip auch ohne einen Referenzsensor, da in Abwesenheit des Analyten oder bei geringen Konzentrationen bspw. im gesunden Zustand ein Referenzsignal im Sensor 14 selbst generiert wird. Hierdurch kann ein Referenzpunkt ermittelt werden, mittels dem ein Sensordrift oder eine allmähliche Sensordegradation (durch Autolyse der Moleküle, Degradation der Messsensorschicht im seFET oder Einflüsse des Körpers auf das Implantat) herausgerechnet werden kann.

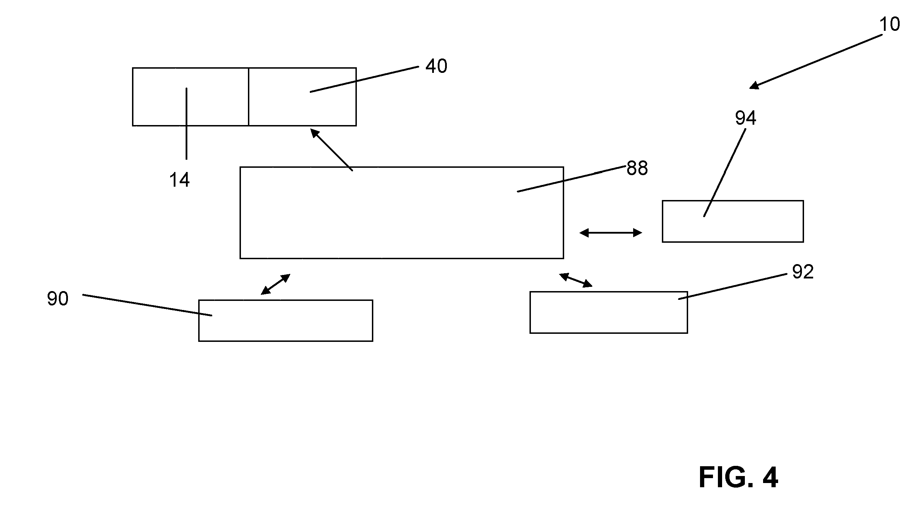

In der Fig. 4 sind schematisch die Bestanteile des Sensorsystems 10 dargestellt. Zusätzlich zu den Sensoren 14, 40 weist das Sensorsystem 10 eine Steuerung 88 mit bspw. Leiterbahnen und/oder weiteren nicht gezeigten Elektronikkomponenten, einen Programmspeicher 90, eine Telemetrieeinheit 92 und eine Stromversorgung 94 auf. Mittels der Telemetrieeinheit 92 können die vom Sensorsystem 10 erfassten Werte an ein (nicht dargestelltes) externes Gerät übertragen werden. Die Telemetrieeinheit 92 ist bevorzugt für bidirektionale Kommunikation ausgeführt, so dass das Sensorsystem 10 durch ein externes Gerät gesteuert werden kann. Weiterhin kann das Sensorsystem 10 mittels der Telemetrieeinheit 92 mit weiteren, implantierten Geräten kommunizieren, um zum Beispiel eine Therapie oder Medikamentenabgabe dieser weiteren implantierten Geräte in Abhängigkeit von den gemessenen Sensorwerten zu steuern.

Alternativ oder zusätzlich kann ein Bereich des Sensorsystems 10 und/oder des Implantats 54 eine Beschichtung bzw. eine Erkennungsbeschichtung 96 aufweisen, die molekulare Erkennungsmarker für Zellen aufweist, wobei die Erkennungsmarker Peptide bzw. Oligopeptide sind. Nach einer Implantation des Sensorsystems 10 im Blutstrom werden durch die spezielle Beschichtung 96 Endothel-Progenitorzellen aus dem Blutstrom und Endothelzellen angelockt, die sich auf der Artikeloberfläche ansiedeln, proliferieren und nach einigen Tagen eine Monoschicht Endothel bilden. Die Endothelzellen wachsen auch über das semipermeable Sensorfenster, bilden aber genügen große Zwischenzell-Poren aus, dass der Analyt 30 bzw. das Cystatin C aus der angrenzenden Körperflüssigkeit in das Probenvolumen 60 diffundieren kann (nicht gezeigt).

Im Folgenden werden drei Beispiele zur Herstellung der Erkennungsbeschichtung 96 beschrieben:

### Beispiel 1 für Oberflächenbeschichtung:

Ein im Sauerstoffplasma oder durch Spülen mit der Lösungsmittelreihe Dichlormethan, Aceton, Methanol und Millipore Wasser gereinigter Sensorkopf wird wie folgt weiterbehandelt:
Es wird eine 1 mM Lösung von Hydroxyundecylphosphonsäure in trockenem Tetrahydrofuran (THF) hergestellt. In diese wird der Sensorkopf gehängt und das Lösungsmittel wird innerhalb von einer Stunde abgedampft, wobei der Meniskus der Lösung über die Sensoroberfläche wandert. Anschließend wird der Sensorkopf 18 Stunden bei 120°C getempert und daraufhin mit Lösungsmittel THF gespült. Die so vorbehandelte Oberfläche wird 15 Stunden in eine 0,3 M Lösung von Carbonyldiimidazol (CDI) in trockenem Dioxan gelegt. Anschließend wird das Substrat zwei mal 10 Minuten mit trockenem Dioxan gespült und anschließend im Stickstoffstrom getrocknet. Auf die so behandelte Oberfläche wird eine Lösung der zu koppelnden Verbindungen (hier ein cyclisches Pentapeptid gemäß Formel II mit y = 2 (ca. 50 µg/ml) in PBS-Puffer (aminosäurefrei) gegeben und über Nacht bei 4°C geschüttelt. Anschließend wird der Sensorkopf mit Puffer gespült.

### Beispiel 2 für Oberflächenbeschichtung:

Ein gemäß Beispiel 1 gereinigtes Sensorgehäuse aus Titan (Ti), das aus einem Zylinder mit Durchmesser 3-7 French besteht, das an einem Kopfende eine Durchführung für ein Sensorkabel und am anderen Ende ein Sensorfenster bestehend aus einer semipermeablen Membran besitzt, wird wie folgt weiterbehandelt:
Es wird eine 3 mM Lösung von 3-(4-Oxybenzophenone)propylphosphonsäure in trockenem Tetrahydrofuran hergestellt. Mit dieser Lösung wird die gereinigte Oberfläche drei Mal besprüht. Anschließend wird das Gehäuse 12 Stunden bei 120°C getempert und daraufhin mit dem Lösungsmittel THF gespült. Das Titangehäuse wird in eine Lösung der zu koppelnden Verbindungen (hier ein cyclisches Pentapeptid gemäß Formel II mit y = 2 (ca.500 µg/ml) in PBS-Puffer gemäß Beispiel 1 gegeben und über Nacht bei 4°C geschüttelt. Am nächsten Tag werden die Ti-Sensoroberflächen aus dem Lösungsmittel entfernt, getrocknet und bei 260 nm mit 100 mW/cm² belichtet. Nichtgebundenes Protein wird abgewaschen.

### Beispiel 3 für Oberflächenbeschichtung:

Die gereinigten Sensorgehäuse aus Titan (siehe Beispiel 2) werden in einer Mischung aus Toluol, Triethylamin und 3-Aminopropyltriethoxysilan gegeben und 14 Std. bei Raumtemperatur inkubiert. Nach Ablauf der Reaktion wird der Sensor in Toluol gewaschen und für 1 Std. bei 135 °C getempert.

Zusammensetzung der Silanisierungslösung: 10 ml Toluol, getrocknet; 0,5 ml Trietylamin; 1 ml Silan 3 Aminopropyltriethoxysilan.

An den Reinigungsschritt (spülen des Ti Substrates mit Trichlormethan) schließt sich die Aktivierung mit 1,1'-Carbonyl Diimidazol (CDI) an. Die silanisierten und gespülten Ti-Substrate werden für 5 Stunden in CDI gegeben. Dazu wird das CDI in trockenem Dioxan gelöst. Hier eignet sich eine Stammlösung von 2,5 g/50 ml CDI in Dioxan, die für mehrere Tage (2 d, trocken) haltbar ist. Die Substrate werden bei Raumtemperatur leicht bewegt. Nach der Aktivierung werden die Substrate entnommen und mit trockenem Dioxan gespült. Für die Ankopplung der cyclischen Peptide gemäß Formel I mit x = 2 werden die aktivierten Ti-Substrate in die Peptid Lösung einer Konzentration von 5 mg/ml, getaucht und bei 4°C über Nacht (min. 12 Std.) gekoppelt. Die Reaktion geschieht geeigneter Weise in 125 mM Natriumborat mit 0,066% SDS bei einem pH-Wert von 10,0. Die Lösung ist danach wieder verwendbar bzw. es können mehrere Oberflächen mit dieser Lösung behandelt werden.

Die Sensoren 14 werden nach der Kopplung dreimal mit 5 ml des Borax-Puffers (oben) gewaschen. Danach noch dreimal mit Wasser. Die nach diesen Waschschritten noch analysierbaren Peptide sind kovalent gebunden.

Die in den Beispielen 1 bis 3 beschichteten implantierbaren Sensoren 14 zeigten allesamt nach Implantation und Einwachsphase eine definierte, einschichtige, zeitlich unveränderliche und nicht-trombogene Vernarbung. Die Sensoren 14 waren allesamt für wenigstens 6 Monate einsatzfähig. Die semipermeablen Membranen 36 der Sensoren 14 blieben für den Analyten 30 in konstanter Weise permeabel, so dass zuverlässige und reproduzierbare Signale erzeugt wurden. Dementsprechend haben erfindungsgemäße implantierbare Implantate 54 allgemein eine Funktionsfähigkeit nach dem Implantieren von wenigstens drei Monaten, bevorzugt wenigstens sechs Monaten und besonders bevorzugt von wenigstens einem Jahr.

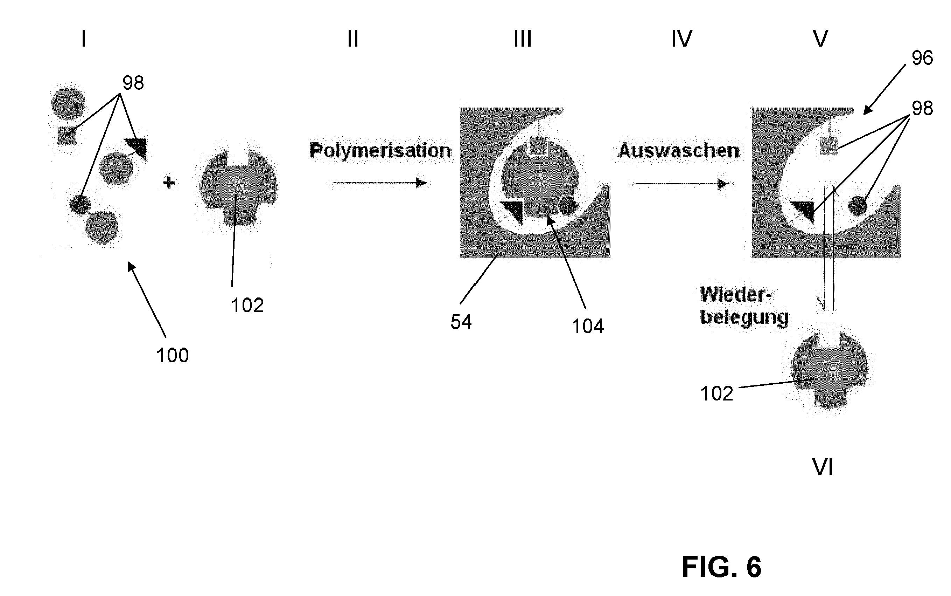

Die Fig. 6 zeigt eine weitere Möglichkeit zur Modifikation der Implantatoberfläche durch Imprinten von spezifischen Erkennungsmarkern, beispielsweise von Polymeren 98. Hierbei wird ein Polymergemisch 100 mit einem Template 102 gemischt (I) und polymerisiert (II). Der resultierende Komplex 104 wird zu dessen Beschichtung auf das Implantat 54 aufgebracht (III). In Anschluss wird das Implantat 54 gewaschen (IV), wobei Polymere 98 des des Polymergemischs 100 als Erkennungsbeschichtung 96 auf dem Implantat 54 verbleiben (V) und das Template 102 zu dessen erneuter Belegung mit Polymergemisch 100 ausgewaschen wird (VI).

Hierdurch kann eine "selbstheilende" Oberfläche mit guter Biokompatibilität erzeugt werden. Durch die auf der Oberfläche der Implantate strukturell angepassten langzeitstabilen Template 102 können spezielle Polymere 98 *in vivo* spezifisch über molekulare Erkennungsevents selektiv und stabil an die Oberfläche zu binden. Eine gute Biokompatibilität kann hierdurch erreicht werden. Andere strukturell zu 98 unterschiedliche Polymere, die z.B. die in Folge der Adhäsion an unmodifizierte Oberflächen die Gerinnungskaskade im Blut auslösen könnten, können aufgrund der abweichenden Struktur nicht oder nur kurz an die Templates 102 binden und werden schnell vom eigentlichen Polymer 98 von den Templates 102 verdrängt. Verliert das Polymer 98 im Template 102 aufgrund chemischer Modifikationen / Degradationen die dreidimensionale Struktur, desorbiert es von der Implantatsoberfläche und schafft hierdurch Platz für Polymere 98 mit korrekter Struktur. Die biokompatible Oberfläche erneuert sich von selbst.

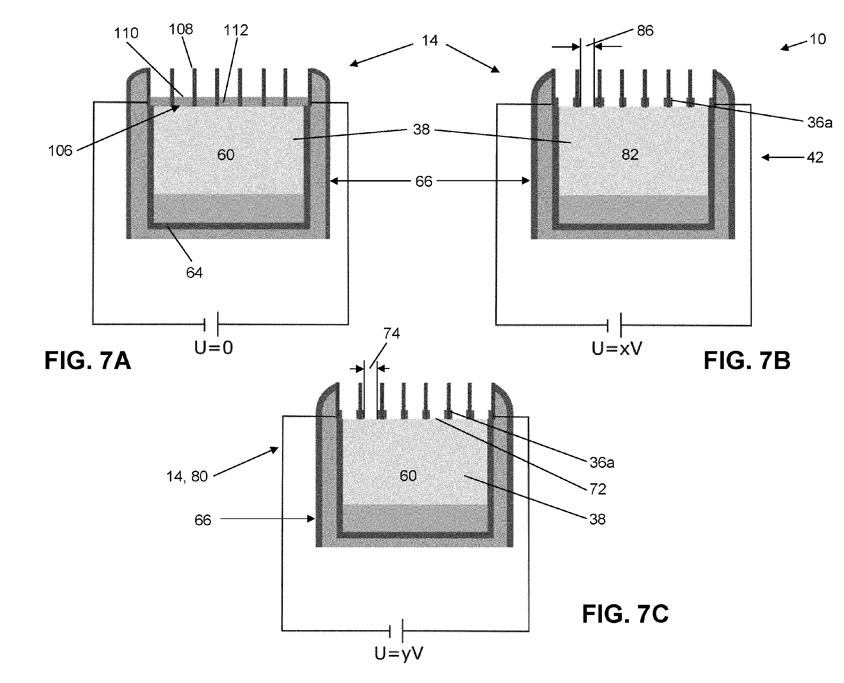

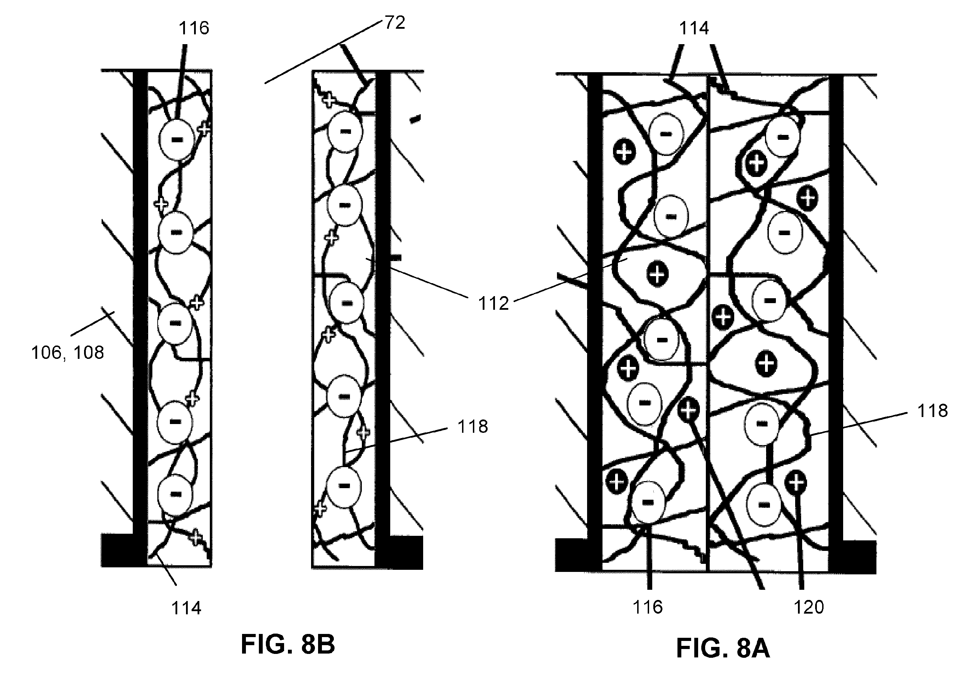

In den Fig. 7A-C, 8A und 8B ist ein alternatives Ausführungsbeispiel des Sensorsystems 10 dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele ist jedoch den Bezugszeichen der geänderten Ausführungen des Ausführungsbeispiels der Fig. 7A-C, 8A und 8B der Buchstabe a hinzugefügt. Die nachfolgende Beschreibung beschränkt sich im Wesentlichen auf die Unterschiede zu dem Ausführungsbeispiel in den Fig. 1 bis 6, wobei bezüglich gleich bleibender Bauteile, Merkmale und Funktionen auf die Beschreibung des Ausführungsbeispiels in den Fig. 1 bis 6 verwiesen werden kann.

In den Fig. 7A bis 7C sind Schnittdarstellungen eines alternativen medizinischen Sensorsystems 10 in drei unterschiedlichen Zuständen (geschlossen, Referenzmessung, Analytmessung) gezeigt. Das Nachweissystem 66 ist nicht im Detail gezeigt. Die Zustände können über eine elektrisch steuerbare Membran 36a eingestellt werden, die über eine Trägerstruktur 106 mit dem Gehäuse 56 verbunden ist. Die Trägerstruktur 106 ist von einem nanoporösen Substrat aus TiO₂ gebildet und weist somit eine hohe Biokompatibilität auf. Die Trägerstruktur 106 ist von sich senkrecht zu dem Boden 64 der Reservoirs 38 und parallel zueinander erstreckenden Nanoröhrchen 108 gebildet. Jedes Nanoröhrchen 108 weist eine für den Analyten 30 permeable Nanopore 110 auf. Für die Messung von Cystatin C ist ein Durchmesser von ca. 10 nm bevorzugt, für die Messung von Glukose ein Durchmesser von ca. 1 nm. Die Innenoberfläche der Nanopore 110 ist an einer zum Boden 64 weisenden Seite mit einem leitfähigen Material in der Form von Gold durch Sputtern beschichtet. Auf der dem Probenvolumen 60 zugewandten Oberfläche ist die steuerbare, organische Membran 36a angeordnet. Die steuerbare, organische Membran 36a wird aus einer Lösung seiner Komponenten auf der Goldoberfläche der Nanoporen 110 elektropolymerisiert. Zudem ist die steuerbare, organische Membran 36a von einem elektroaktiven Material bzw. Polymer 112 gebildet, das Polypyrrol (PPy) 114 und Dodecylbenzolsulfonsäure (DBS) 116 aufweist (vgl. Fig. 8). Zu einer Steuerung der steuerbaren, organischen Membran 36a sind hier nicht näher gezeigte Leiterbahnen an die Trägerstruktur 106 auf der Höhe der Goldbeschichtung angebracht und mit einer im Sensor 14 integrierten Steuerung 88 verbunden, wodurch die steuerbare, organische Membran 36a elektrisch steuerbar ist.

Bei einer Herstellung der Trägerstruktur 106 bzw. der Nanoröhrchen 108 kann der Porendurchmesser leicht eingestellt werden, wodurch, abgestimmt auf den einzusetzenden Analyten 30, eine Vielzahl von unterschiedlichen Trägerstrukturen zur Verfügung gestellt werden können, die Grundgerüste für die steuerbare, organische Membran 36a bilden. Eine Schichtdicke der Membran 36a ist hierbei deutlich größer (beispielsweise mehrere 100 µm) als ein Durchmesser der Nanoröhrchen 108.

Durch das elektroaktive Polymer 112 weist die steuerbare, organische Membran 36a ein Material auf, dessen Redoxzustand änderbar ist. Somit können über Kontakte zwischen dem leitfähigen Material und der Steuerung 88 die Redoxzustände und damit ein Volumen des elektroaktiven Polymers 112 bzw. der steuerbaren, organischen Membran 36a verändert bzw. gesteuert werden. Die Vergrößerung des Volumens führt zum vollständigen Verschluss der Nanoporen 110 der Trägerstruktur 106 und der Poren 72 der steuerbaren, organischen Membran 36a, umgekehrt bewirkt die Verkleinerung des Volumens die Öffnung der Nanoporen 110 und der Poren 72 für den Analyten 30. Weil die Reduktion bzw. Oxidation des elektroaktiven Polymers 112 partiell erfolgen kann, ist auch die Öffnung der Nanoporen 110 und der Poren 72 entsprechend partiell und stufenlos regulierbar, was wiederum eine Abstimmung an verschiedene Analyten 30 möglich macht.

Die Fig. 8A und 8B zeigen eine Pore 72 in einem geschlossenen (Fig. 8A) und in einem offenen Zustand (Fig. 8B). Das elektroaktive Polymer 112 besteht aus einer Matrix 118 quervernetzten positiv geladener Fasern aus Polypyrrol 114. Während der Polymerisation bei der Beschichtung der Goldschicht lagern sich in diese Matrix 118 negativ geladene DBS-Moleküle 116 ein, die aufgrund ihrer Größe nicht aus der Matrix 118 diffundieren können und die negativ geladenen Gegenionen zur positiv geladenen Matrix 118 aus Polypyrrol 114 darstellen. Bei einer vollständigen Reduktion des Polypyrrols 114 wird dieses elektrisch neutral.

Ein Schließen der Poren 72 erfolgt folgendermaßen: Um die negative Ladung der DBS-Moleküle 116 zu kompensieren, werden durch Anlegen einer Spannung von beispielsweise 2 Volt positiv geladene, hydratisierte Natriumionen 120 in die Matrix 118 eingelagert. Dort führen sie zu einer starken (bis zu 30%) lateralen Volumenänderung des elektroaktiven Polymers 112. Diese Änderung des Volumens führt zu einem Verschluss der Poren 72 und verhindert den Eintritt von Strukturen in das Probenvolumen 60. Eine Reversion des Vorganges erfolgt durch Anlegen einer Spannung umgekehrter Polarität, die dann wieder zu einer Volumenreduktion des Polymers 112 führt. Die Reversibilität dieses Vorganges ermöglicht ein wiederholbares Öffnen und Schließen der Poren 72. Ferner ist über den Grad der Reduktion des Volumens des Polymers 112 eine partielle Volumenänderung der steuerbaren, organischen Membran 36a möglich. Die jeweiligen Redoxzustände des elektroaktiven Polymers 112 werden über unterschiedliche angelegte Spannungen erzeugt und können bei einer Abschaltung der Spannung beibehalten werden.

Durch die steuerbare Membran 36a, die zwischen einem geöffneten und einem geschlossenen Zustand des Reservoirs 38 reversibel veränderbar ist, kann der Sensor 14 sowohl Messsensor 80 wie auch Referenzsensor 42 sein. Hierbei ist die Membran, wie in Fig 7A gezeigt, vor einer ersten Messung des Sensorsystems 10, aber auch zwischen den verschiedenen Messungen geschlossen, so dass in diesem Zustand weder eine Struktur noch das Merkmal 12 bzw. der Analyt 30 in das Probevolumen 60 eindringen noch aus diesem austreten kann.

Zum Nachweis des Merkmals 12 wird hier in einem ersten Schritt ein erster Durchmesser 86 der Pore 72 der steuerbaren, organischen Membran 36a eingestellt (vgl. Fig. 7B) und in einem zweiten Schritt ein zweiter Durchmesser 74 der Pore 72 eingestellt (vgl. Fig. 7C), wobei der erste Durchmesser 86 kleiner als der zweite Durchmesser 74 ist. Der erste Schritt stellt eine Referenzmessung dar, bei der so viele Störsignale wie möglichst ermittelt werden sollen. Der erste Durchmesser 86 ist gerade so eingestellt, dass das Merkmal 12 bzw. der Analyt 30 nicht in das Probenvolumen 60 eindringen kann und beträgt beispielsweise ca. 5 nm. Alle kleineren Moleküle 78, die eine Bestimmung des Analyten 30 beeinträchtigen könnten, können hingegen eindringen. Der zweite Schritt ist eine Analytmessung, hier wird der zweiter Durchmesser 74 gerade so weit auf beispielsweise ca. 10 nm für die Messung von Cystatin C oder auf 1 nm für die Messung von Glukose vergrößert, dass der Analyt 30 nun in den Probenvolumen 60 eindringen kann, um gemessen zu werden. Dies kann durch ein Anlegen unterschiedlicher Spannungen von beispielsweise 1 V bei der Referenzmessung und einer Spannung von 1,5 V bei der Analytmessung erfolgen. Alternativ kann der Durchmesser bei konstant angelegter Spannung (beispielsweise 2 V) durch die Dauer der angelegten Spannung erfolgen. Typische Werte sind 4 Minuten für die Referenzmessung und 5 Minuten bei der Analytmessung. Zum Erhalten eines Endmessergebnisses der Konzentration an Analyt 30 wird nun das Messergebnis der Analytmessung um das Messergebnis der Referenzmessung korrigiert.

### Bezugszeichenliste

- 10: Sensorsystem
- 12: Merkmal
- 14: Sensor
- 16: Merkmalsträger
- 18: Merkmalsträgerrezeptor
- 20: Merkmalsträger
- 22: Halbleiterbauelement
- 24: Bereich
- 26: Antigenerkennungsstelle
- 28: Antigen
- 30: Analyten
- 32: Antagonist
- 34: Ladung
- 36: Membran
- 38: Reservoirs
- 40: Sensor
- 42: Referenzsensor
- 44: Passivierungsschicht
- 46: Bindungsstelle
- 48: Oberflächenbeschichtung
- 50: Bereich
- 52: Störstoff
- 54: Implantat
- 56: Gehäuse
- 58: Sensorarray
- 60: Probenvolumen
- 62: Seite
- 64: Boden
- 66: Nachweissystem
- 68: Selektionsstruktur
- 70: Seite
- 72: Pore
- 74: Durchmesser
- 76: Molekül/Zelle
- 78: Kleinstmolekül
- 80: Messsensor
- 82: Referenzvolumen
- 84: Pore
- 86: Durchmesser
- 88: Steuerung
- 90: Programmspeicher
- 92: Telemetrieeinheit
- 94: Stromversorgung
- 96: Erkennungsbeschichtung
- 98: Polymer
- 100: Polymergemisch
- 102: Template
- 104: Komplex
- 106: Trägerstruktur
- 108: Nanoröhrchen
- 110: Nanopore
- 112: Polymer
- 114: Polypyrrol
- 116: Dodecylbenzolsulfonsäure
- 118: Matrix
- 120: Natriumion

## Patentansprüche

1. Medizinisches Sensorsystem (10, 10') zum Nachweis von zumindest einem Merkmal (12) eines tierischen und/oder menschlichen Körpers, mit zumindest einem Sensor (14), einem ersten Merkmalsträger (16) und einem Merkmalsträgerrezeptor (18), wobei sich der erste Merkmalsträger (16) zumindest in einem Merkmalsparameter von einem zumindest zum Zeitpunkt des Nachweises vorhandenen zweiten Merkmalsträger (20) unterscheidet, **dadurch gekennzeichnet, dass** der zumindest eine Sensor (14) bei dem Nachweis des zumindest einen Merkmals *in vivo* angeordnet ist.

2. Medizinisches Sensorsystem nach Anspruch 1, **dadurch gekennzeichnet, dass** der Sensor (14) zumindest eine elektrische Zustandsgröße ermittelt.

3. Medizinisches Sensorsystem nach Anspruch 1 oder 2, **dadurch gekennzeichnet, dass** der Sensor (14) zumindest eine Spannungsänderung ermittelt.

4. Medizinisches Sensorsystem nach einem der vorhergehenden Ansprüche, **dadurch gekennzeichnet, dass** der Sensor (14) zumindest ein Halbleiterbauelement (22) aufweist.

5. Medizinisches Sensorsystem nach einem der vorhergehenden Ansprüche, **dadurch gekennzeichnet, dass** zumindest ein Bereich (24) des Sensors (14) mit zumindest dem Merkmalsträgerrezeptor (18) beschichtet ist.

6. Medizinisches Sensorsystem nach einem der vorhergehenden Ansprüche, **dadurch gekennzeichnet, dass** der Merkmalsträgerrezeptor (18) von einem Molekül gebildet ist, das eine Antigenerkennungsstelle (26) aufweist.

7. Medizinisches Sensorsystem nach einem der vorhergehenden Ansprüche, **dadurch gekennzeichnet, dass** der zweite Merkmalsträger (20) von einem ein Antigen (28) aufweisenden Analyten (30) und/oder der erste Merkmalsträger (16) von einem Antagonist (32) des Analyten (30) gebildet ist.

8. Medizinisches Sensorsystem nach einem der vorhergehenden Ansprüche, **dadurch gekennzeichnet, dass** der erste Merkmalsträger (16) eine höhere Ladung (34) wie der zweite Merkmalsträger (20) aufweist.

9. Medizinisches Sensorsystem nach einem der vorhergehenden Ansprüche, **gekennzeichnet durch** zumindest eine organische Membran (36, 36a), mittels der zumindest ein Reservoir (38) des Sensors (14) abschließbar ist, wobei die organische Membran (36, 36a) so ausgebildet ist, dass der erste Merkmalsträger (16) zu jeder Zeit im Reservoir (38) zurückhaltbar ist und/oder dass sie zumindest zeitweise für den zweiten Merkmalsträger (20) passierbar ist.

10. Medizinisches Sensorsystem nach Anspruch 9, **dadurch gekennzeichnet, dass** die organische Membran (36a) steuerbar, insbesondere elektrisch steuerbar, ausgeführt ist.

11. Medizinisches Sensorsystem nach einem der vorhergehenden Ansprüche, **gekennzeichnet durch** zumindest einen zweiten Sensor (40), der als Referenzsensor (44) ausgebildet ist.

12. Medizinisches Sensorsystem nach einem der vorhergehenden Ansprüche, **gekennzeichnet durch** zumindest eine Passivierungsschicht (44), welche auf zumindest einem Bereich (24) des Sensors (14) aufgebracht ist und dazu ausgebildet ist unspezifische Bindungsstellen (46) abzusättigen.

13. Medizinisches Sensorsystem nach einem der vorhergehenden Ansprüche, **gekennzeichnet durch** zumindest eine Oberflächenbeschichtung (48), welche auf zumindest einem Bereich (50) des Sensors (14) aufgebracht ist und dazu ausgebildet ist Wechselwirkungen mit Störstoffen (52) zu verhindern.

14. Medizinisches Implantat (54) mit zumindest einem medizinischen Sensorsystem (10, 10') nach einem der Ansprüche 1 bis 13.

15. Verfahren zum Betreiben eines medizinischen Sensorsystems (10, 10') zum Nachweis von zumindest einem Merkmal (12) eines tierischen und/oder menschlichen Körpers mit zumindest einem Sensor(14), einem ersten Merkmalsträger (16) und einem Merkmalsträgerrezeptor (18), wobei sich der erste Merkmalsträger (16) zumindest in einem Merkmalsparameter von einem zumindest zum Zeitpunkt des Nachweises vorhandenen zweiten Merkmalsträger (20) unterscheidet, **dadurch gekennzeichnet, dass** der zumindest eine Sensor (14) bei dem Nachweis des zumindest einen Merkmals (12) *in vivo* angeordnet wird und vom Sensor (14) im Nachweisprozess eine Merkmalsänderung ermittelt wird, welche durch eine reversible Verdrängung des ersten Merkmalsträgers (16) vom Merkmalsträgerrezeptor (18) durch den zweiten Merkmalsträger (20) hervorgerufen wird.
